# EUROPEAN PATENT APPLICATION

(11) **EP 4 692 047 A1**
(43) Date of publication of application: **11.02.2026**
(21) Application number: 24780140.0
(22) Date of filing: 25.03.2024
(51) Int. Cl.: C07C 229/18, C07D 295/112, C07D 295/155, G01N 21/64, G01N 33/48

(54) **COMPOUND, FLUORESCENT DYE, KIT, CELL DETECTION METHOD, AND DYEING MATERIAL**

(30) Priority: 24.03.2023 JP 2023047897
(71) Applicant: National University Corporation Kochi University, Kochi-shi, Kochi 780-8520 (JP); National University Corporation Ehime University, Ehime 790-8577 (JP)
(72) Inventor: NIKO, Yosuke, Kochi-shi, Kochi 780-8520 (JP); TOUCHI, Yuki, Kochi-shi, Kochi 780-8520 (JP); HASHIMOTO, Takuya, Kochi-shi, Kochi 780-8520 (JP); YAMAMOTO, Riko, Kochi-shi, Kochi 780-8520 (JP); MURAKAMI, Masamoto, Toon-shi, Ehime 791-0295 (JP); KAWAKAMI, Ryosuke, Toon-shi, Ehime 791-0295 (JP); TSUDA, Teruko, Toon-shi, Ehime 791-0295 (JP); IMAMURA, Takeshi, Toon-shi, Ehime 791-0295 (JP)
(74) Representative: Cabinet Beau de Loménie
(86) International application number: PCT/JP2024/011617
(87) International publication number: WO 2024/204022

(57) **Abstract**

Problem to be Solved

It is an object of the present invention to provide a compound suitably usable in fluorescent dye agents, a novel fluorescent dye agent and kit for conveniently detecting cells, a novel method for conveniently detecting cells, and staining materials.

Solution to the Problem

A compound represented by the following formula (1) or a salt thereof: 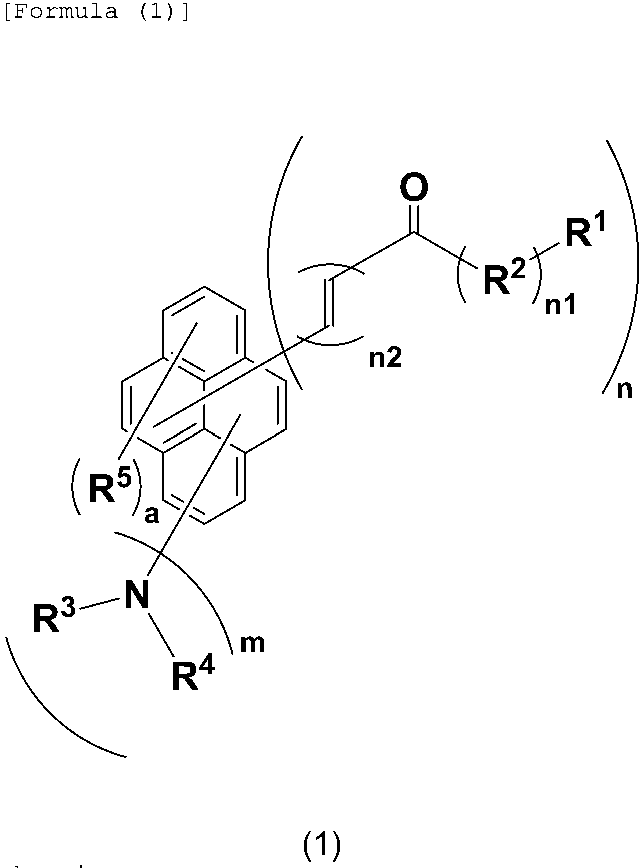 wherein,
R¹ is a substituted or unsubstituted C1-12 alkyl group, C2-12 alkenyl group, or C2-12 alkynyl group, etc.

## Description

### Technical Field

The present invention relates to a fluorescent dye for use in detecting cells, a compound usable therein, a kit, a method for detecting cells, and a material for staining, among others.

### Background Art

In the detection of cells and tissues, for example, in the detection of tumor cells, various staining methods have been employed. For instance, hematoxylin and eosin (HE) staining is the most widely used method in pathological diagnosis and is regarded as the gold standard in many diagnostic procedures. However, in HE staining, normal tissues are also stained in addition to tumor cells, resulting in poor light transmittance, thereby requiring thin-sectioning of tissue specimens. Since thin sections provide only two-dimensional information, in order to determine the distribution range of tumor cells, it is necessary to prepare and observe a large number of sections. Moreover, the preparation of tissue specimens requires advanced skills and is a complicated and time-consuming process.

Furthermore, in HE staining, there are cases where the boundary between tumor cells and normal tissues becomes low-contrast and indistinct.

In addition, methods are known in which specific tissues are stained using antibodies conjugated with fluorescent dyes, radionuclides, metal particles, and the like, to obtain high-contrast images of tumor cells. However, since the antibody moiety does not penetrate the cell membrane, treatment for increasing membrane permeability is required, which complicates the operation. Furthermore, such reagents are expensive.

On the other hand, methods have been reported in which abnormal tissues and normal tissues are irradiated with light without using staining dyes, and the differences in Raman scattering are utilized to detect skin diseases (see, for example, Patent Document 1). However, it is presumed that the Raman scattering method makes it difficult to determine the tumor region at the cellular level.

### Prior Art Documents

### Patent Documents

Patent Document 1: JP-A-2018-201678

### Summary of Invention

### Problems to be Solved by the Invention

It is an object of the present invention to provide a compound suitable for use in a fluorescent dye or the like.

It is another object of the present invention to provide a novel fluorescent dye and kit for easily detecting cells or the like.

It is another object of the present invention to provide a novel method for easily detecting cells or the like.

It is still another object of the present invention to provide a novel staining material for lipid bilayers, oil droplets, protein aggregates, or the like, which are present in cells or model cellular environments.

### Means for Solving the Problems

The present inventors have newly created compounds exhibiting novel solvatochromism, and found that fluorescent dyes containing such compounds, when applied to staining of cells and tissues, allow cells and the like to be easily detected, thereby accomplishing the present invention.

Accordingly, the present invention provides the following compounds or salts thereof.
[1] A compound represented by the following Formula (1) or a salt thereof. 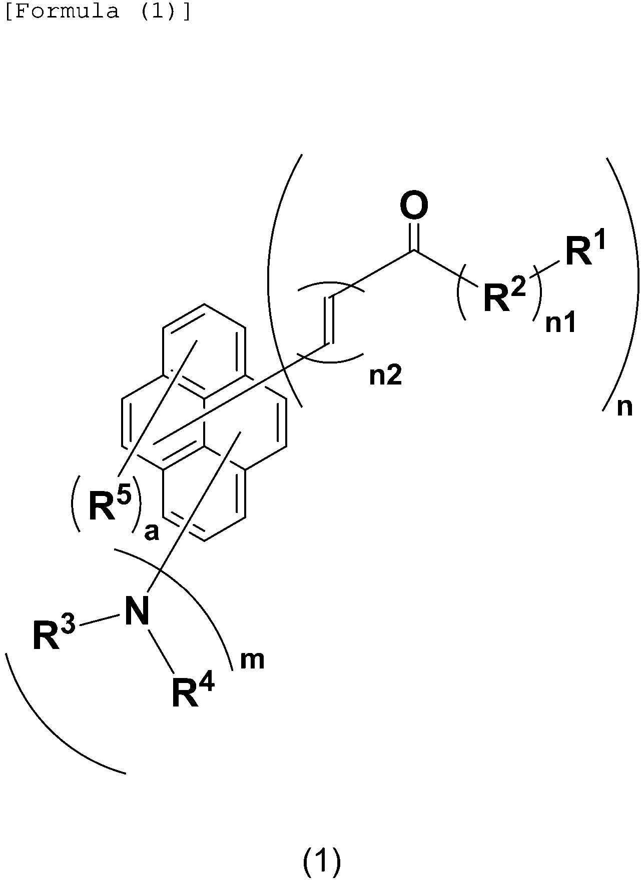 (wherein,
   R¹ is a substituted or unsubstituted C1-12 alkyl group, a C2-12 alkenyl group, or a C2-12 alkynyl group;
   R² is a substituted or unsubstituted methylene group,
   wherein when a plurality of R² groups are present, they may be the same or different;
   n¹ is an integer of 0-4, wherein when n¹ is 2 or more, the respective groups may be the same or different;
   n is an integer of 1-4, wherein when n is 2 or more, the respective groups may be the same or different;
   n² is an integer of 0-4, wherein when n² is 2 or more, the respective groups may be the same or different;
   R³ and R⁴ are each independently selected from hydrogen atom, or a substituted or unsubstituted C1-6 alkyl group, C2-6 alkenyl group, or C2-6 alkynyl group, and R³ and R⁴ may together form a cyclic structure;
   at least one of R¹, R³, or R⁴ includes at least one hydrophilic substituent selected from the group consisting of carboxyl group, hydroxyl group, sulfo group, amino group, amido group, halogen atom, and salts thereof, wherein when two or more hydrophilic substituents are present, the substituents may be the same or different;
   m is an integer of 1-4, wherein when m is 2 or more, the respective groups may be the same or different;
   R⁵ is each independently a C1-12 alkyl group, a C2-12 alkenyl group, a C2-12 alkynyl group, a C5-12 aryl group, a C5-12 heteroaryl group, a halogen atom, or a hydrophilic substituent comprising at least one atom selected from the group consisting of boron, nitrogen, oxygen, phosphorus, sulfur, and halogen; and
   a is each independently an integer of 0-4, wherein when a is 2 or more, the respective R⁵ groups may be the same or different.)
[2] The compound or a salt thereof according to [1], wherein at least one R¹ is selected from a methyl group, an ethyl group, an n-propyl group, an i-propyl group, an n-butyl group, an i-butyl group, a sec-butyl group, a t-butyl group, an ethynyl group, a propargyl group, or a substituted or unsubstituted 1,2,3-triazole or 1,2,4-triazole group having a C1-4 alkyl group.
[3] The compound or a salt thereof according to [1] or [2], wherein R³ and R⁴ are each independently selected from hydrogen atom, a carboxyl group, a hydroxyl group, a sulfo group, an amino group, an amido group, a halogen atom, or a substituted or unsubstituted methyl group, ethyl group, n-propyl group, i-propyl group, n-butyl group, i-butyl group, sec-butyl group, or t-butyl group.
[4] The compound or a salt thereof according to any one of [1]-[3], wherein at least one pair of R³ and R⁴ together form a pyrrolidine ring or a piperidine ring.
[5] The compound or a salt thereof according to any one of [1]-[4], wherein n is 1 or 2.
[6] The compound or a salt thereof according to any one of [1]-[5], wherein n¹ is 1 or 2.
[7] The compound or a salt thereof according to any one of [1]-[6], wherein m is 1 or 2.
[8] The compound or a salt thereof according to any one of [1]-[6], wherein R⁵ is each independently selected from a methyl group, an ethyl group, a hydroxyl group, or an amino group.
[9] The compound or a salt thereof according to any one of [1]-[7], wherein the maximum absorption wavelength is 300-550 nm in 20 mM phosphate buffer (pH 7.4) at 25 °C.
[10] The compound or a salt thereof according to any one of [1]-[8], wherein the molecular weight is 700 or less. The present invention further provides the following fluorescent dyes or the like.
[11] A fluorescent dye comprising the compound or a salt thereof according to any one of [1]-[10].
[12] The fluorescent dye according to [11], for morphological detection of cells and/or tissues.
[13] The fluorescent dye according to [11], for morphological detection of tumor cells.
[14] The fluorescent dye according to [11], for morphological detection of skin cancer cells.
[15] The fluorescent dye according to [11], for staining or visualization of biological samples.
[16] The fluorescent dye according to [11], for fluorescent imaging.
[17] The fluorescent dye according to [11], for in vivo observation.
[18] The fluorescent dye according to [11], for transdermal absorption. The present invention further provides the following kit.
[19] A kit comprising the compound or a salt thereof according to any one of [1]-[10].
   The present invention further provides the following methods for detecting cells.
[20] A method for detecting cells, comprising a step (1) of staining cells with the fluorescent dye comprising the compound or a salt thereof according to any one of [1]-[10].
[21] The method for detecting cells according to [20], further comprising a step (2) of evaluation using fluorescence spectra measured in two or more solvents after step (1).
[22] The method for detecting cells according to [21], wherein step (2) is performed by fluorescent imaging.
   The present invention further provides the following staining material.
[23] A staining material for lipid bilayers, oil droplets, or protein aggregates present in cells or modeling a cellular environment, comprising the compound or a salt thereof according to any one of [1]-[10].

### Effect of the Invention

The compounds of the present invention can be suitably used in fluorescent dyes or the like.

Furthermore, by using the fluorescent dyes and kits of the present invention, cells and the like can be stained clearly, easily, and rapidly.

Furthermore, by using the method for detecting cells of the present invention, lipid bilayers, oil droplets, protein aggregates, or the like, which are present in cells or modeling a cellular environment, can be stained clearly, easily, and suitably.

Furthermore, by using the staining materials of the present invention, lipid bilayers, oil droplets, protein aggregates, or the like, which are present in cells or modeling a cellular environment, can be stained clearly, easily, and rapidly.

### Brief Description of the Drawings

FIG. 1 is a chart showing the ¹H NMR spectrum (500 MHz, CDCl₃) of a mixture of Compound 1a and Compound 1b in the Examples.
FIG. 2 is a chart showing the ¹H NMR spectrum (500 MHz, CDCl₃) of Compound 2a in the Examples.
FIG. 3 is a chart showing the ¹H NMR spectrum (500 MHz, CDCl₃) of Compound 2b in the Examples.
FIG. 4 is a chart showing the ¹H NMR spectrum (500 MHz, DMSO-d₆) of Compound A in the Examples.
FIG. 5 is a chart showing the ¹H NMR spectrum (500 MHz, DMSO-d₆) of Compound B in the Examples.
FIG. 6 is a chart showing the absorption spectrum (left) and fluorescence spectrum (right) of Compound A in organic solvents in the Examples.
FIG. 7 is a chart showing the absorption spectrum (left) and fluorescence spectrum (right) of Compound B in organic solvents in the Examples.
FIG. 8 shows the fluorescence spectra of Compounds A (left) and B (right) in the presence of artificial lipid bilayers (liposomes) in the Examples.
   Green: fluorescence spectrum in the presence of liposomes forming an Lo phase composed of SM/Chol.
   Red: fluorescence spectrum in the presence of liposomes forming an Ld phase composed of DOPC.
   Light blue: phosphate buffer (20 mM, pH = 7.2).
   Lipid concentration: 200 µM; dye concentration: 2 µM; excitation wavelength: 400 nm.

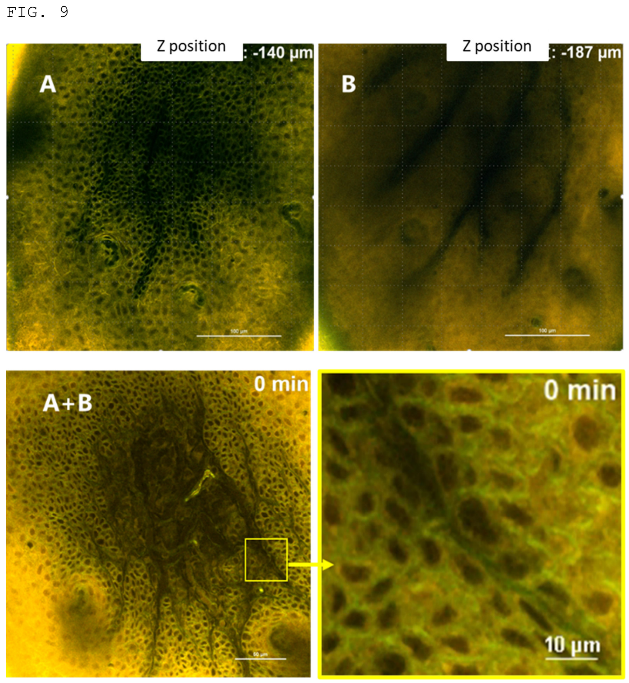

FIG. 9 is a multiphoton fluorescence imaging image of mouse skin tissue (foot) in vivo (spinous layer, basal layer-dermis region). Detection: -492 nm: cyan (second harmonic generation); 500-550 nm: green; 560-593 nm: orange; 593 nm-: red.

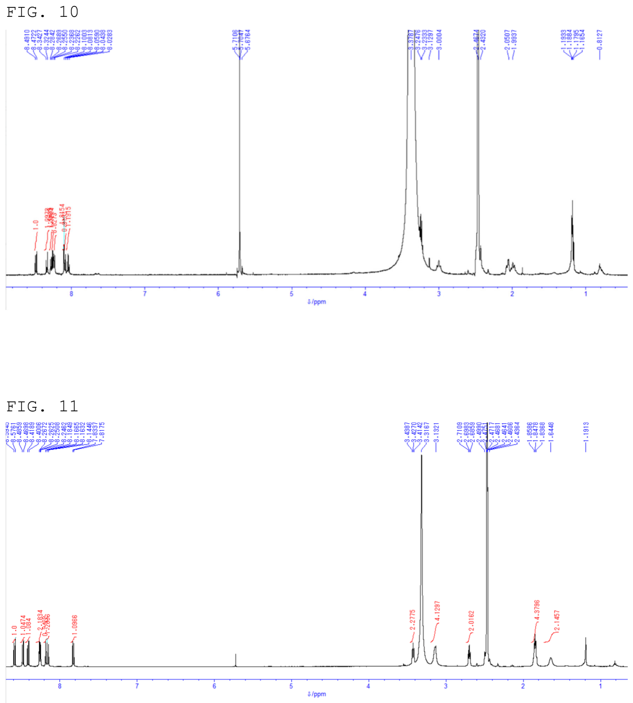

FIG. 10 is a chart showing the ¹H NMR spectrum (500 MHz, DMSO-d₆) of Compound C in the Examples.
FIG. 11 is a chart showing the ¹H NMR spectrum (500 MHz, DMSO-d₆) of Compound D in the Examples.

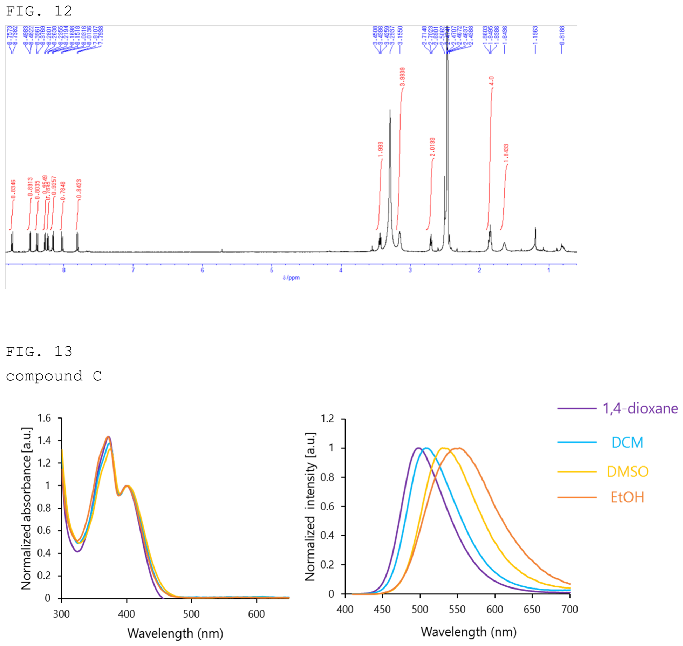

FIG. 12 is a chart showing the ¹H NMR spectrum (500 MHz, DMSO-d₆) of Compound E in the Examples.
FIG. 13 is a chart showing the absorption spectrum (left) and fluorescence spectrum (right) of Compound C in organic solvents in the Examples.

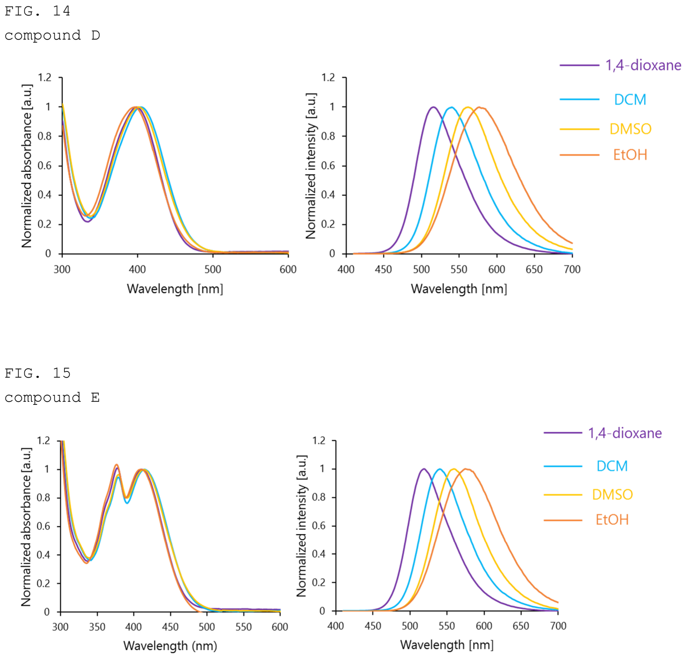

FIG. 14 is a chart showing the absorption spectrum (left) and fluorescence spectrum (right) of Compound D in organic solvents in the Examples.
FIG. 15 is a chart showing the absorption spectrum (left) and fluorescence spectrum (right) of Compound E in organic solvents in the Examples.

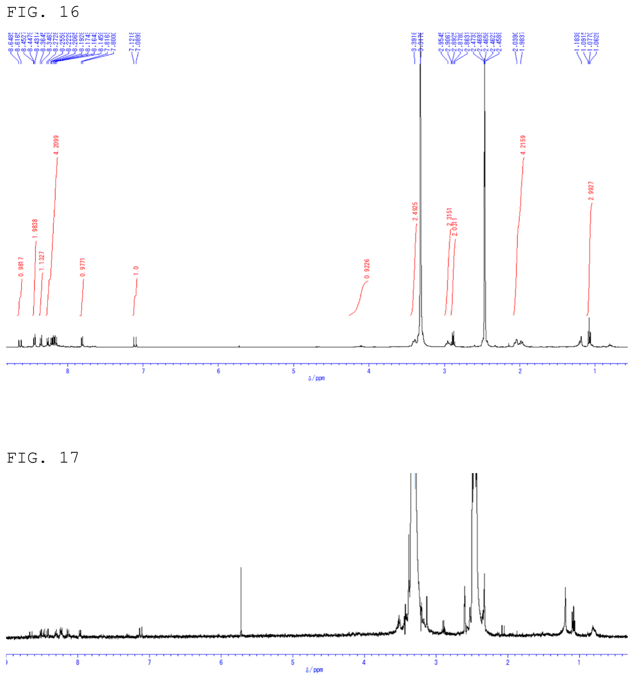

FIG. 16 is a chart showing the ¹H NMR spectrum (500 MHz, DMSO-d₆) of Compound F in the Examples.
FIG. 17 is a chart showing the ¹H NMR spectrum (500 MHz, DMSO-d₆) of Compound G in the Examples.

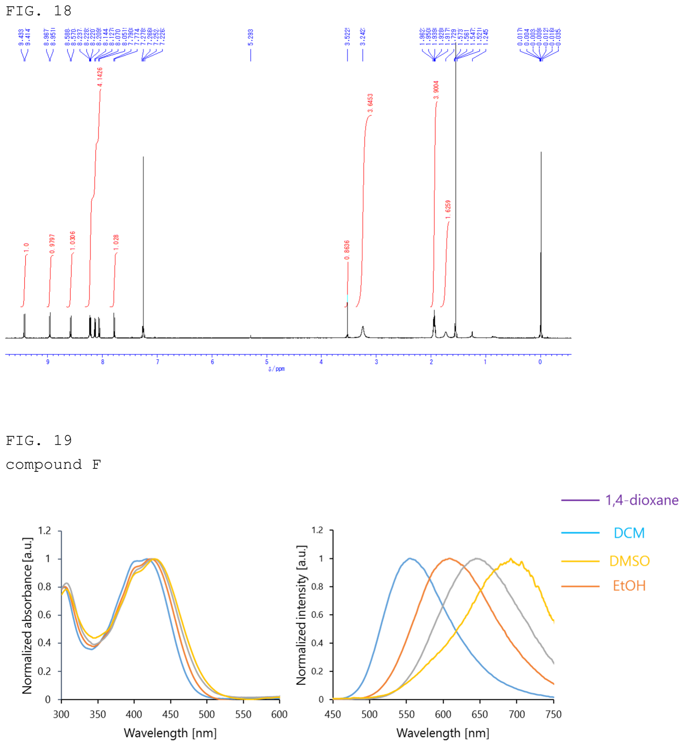

FIG. 18 is a chart showing the ¹H NMR spectrum (500 MHz, CDCl₃) of Compound H in the Examples.
FIG. 19 is a chart showing the absorption spectrum (left) and fluorescence spectrum (right) of Compound F in organic solvents in the Examples.

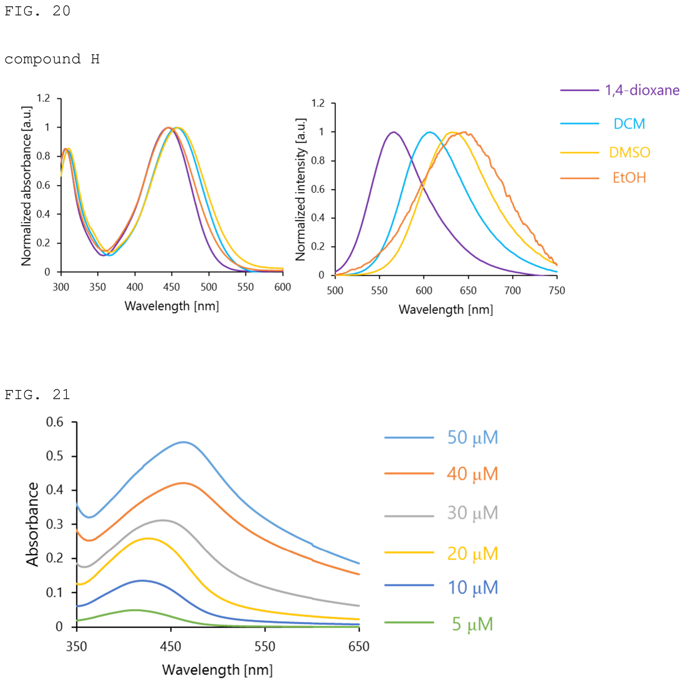

FIG. 20 is a chart showing the absorption spectrum (left) and fluorescence spectrum (right) of Compound H in organic solvents in the Examples.
FIG. 21 is a chart showing the absorption spectrum of PK (an analog of Compound A) in phosphate buffer in the Examples.

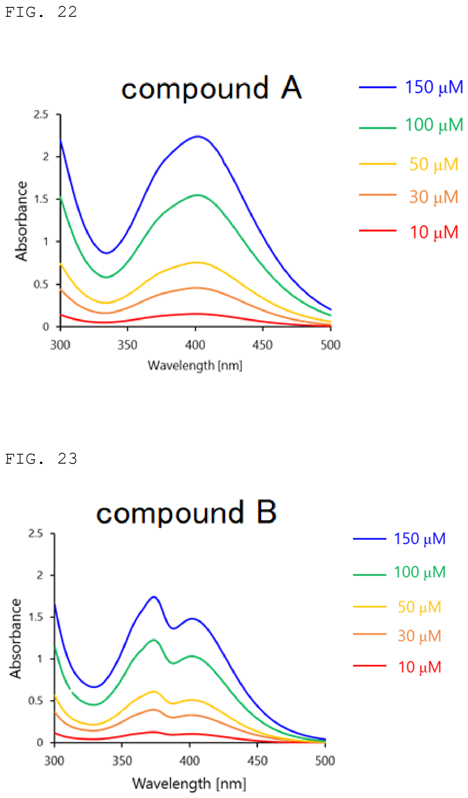

FIG. 22 is a chart showing the absorption spectrum of Compound A in phosphate buffer in the Examples.
FIG. 23 is a chart showing the absorption spectrum of Compound B in phosphate buffer in the Examples.

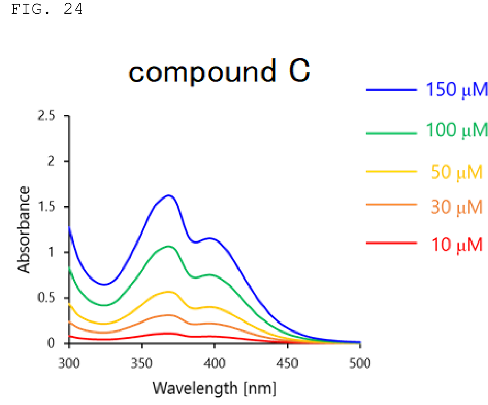

FIG. 24 is a chart showing the absorption spectrum of Compound C in phosphate buffer in the Examples.

### Detailed Description of Embodiments

Hereinafter, embodiments of the present invention will be described in detail; however, the present invention is not limited to these embodiments.

### [Compound]

The compound of the present invention is a compound represented by the following Formula (1) (hereinafter also referred to as "Compound (1)") or a salt thereof. 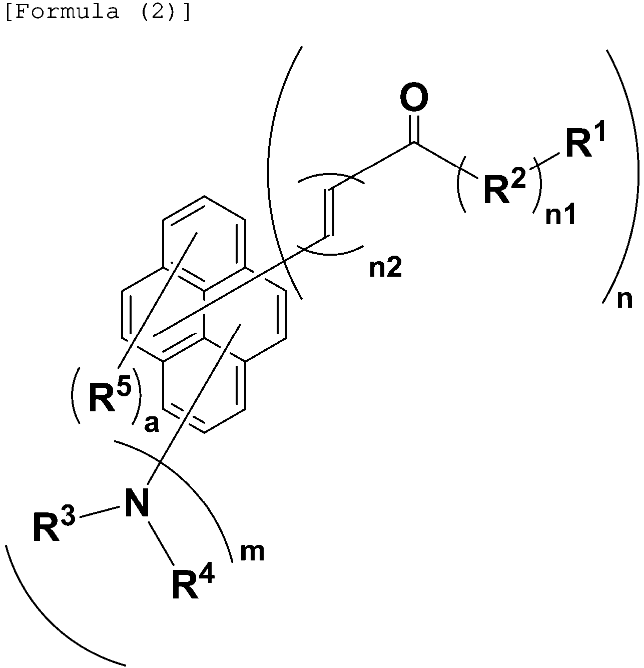 (wherein,
R¹ is a substituted or unsubstituted C1-12 alkyl group, a C2-12 alkenyl group, a C2-12 alkynyl group;
R² is a substituted or unsubstituted methylene group,
wherein when a plurality of R² groups are present, they may be the same or different;
n¹ is an integer of 0-4, wherein when n¹ is 2 or more, the respective groups may be the same or different;
n is an integer of 1-4, wherein when n is 2 or more, the respective groups may be the same or different;
n² is an integer of 0-4, wherein when n² is 2 or more, the respective groups may be the same or different;
R³ and R⁴ are each independently selected from hydrogen atom, or a substituted or unsubstituted C1-6 alkyl group, C2-6 alkenyl group, or C2-6 alkynyl group, and R³ and R⁴ may together form a cyclic structure;
at least one of R¹, R³, or R⁴ includes at least one hydrophilic substituent selected from the group consisting of carboxyl group, hydroxyl group, sulfo group, amino group, amido group, halogen atom, and salts thereof, wherein when two or more hydrophilic substituents are present, the substituents may be the same or different;
m is an integer of 1-4, wherein when m is 2 or more, the respective groups may be the same or different;
R⁵ is each independently a C1-12 alkyl group, a C2-12 alkenyl group, a C2-12 alkynyl group, a C5-12 aryl group, a C5-12 heteroaryl group, a halogen atom, or a hydrophilic substituent comprising at least one atom selected from the group consisting of boron, nitrogen, oxygen, phosphorus, sulfur, and halogen; and
a is each independently an integer of 0-4, wherein when a is 2 or more, the respective R⁵ groups may be the same or different.)

The above Compound (1) may be a compound exhibiting solvatochromism and can be suitably used, for example, in fluorescent dyes or the like. In the present specification, the term "compound exhibiting solvatochromism" refers to a compound in which the maximum absorption wavelength and/or maximum fluorescence wavelength changes depending on the change in polarity (hydrophobicity) of the surrounding environment.

The above compound also includes derivatives such as substituted derivatives in which part or all of the structure is substituted, as well as solvates such as hydrates.

In Formula (1), R¹ is a substituted or unsubstituted C1-12 alkyl group, a C2-12 alkenyl group, or a C2-12 alkynyl group.

Examples of the C1-12 alkyl group represented by R¹ include straight-chain or branched alkyl groups such as methyl, ethyl, n-propyl, isopropyl, n-butyl, iso-butyl, sec-butyl, tert-butyl, pentyl, isopentyl, neopentyl, n-hexyl, n-heptyl, n-octyl, n-nonyl, n-decyl, n-undecyl, and n-dodecyl groups.

Examples of the C2-12 alkenyl group represented by R¹ include straight-chain or branched alkenyl groups such as vinyl and allyl groups.

Examples of the C2-12 alkynyl group represented by R¹ include straight-chain or branched alkynyl groups such as ethynyl and propargyl groups.

Examples of substituents of the alkyl group and the like represented by R¹ include carboxyl group, hydroxyl group, sulfo group, amino group, amido group, halogen atom, and groups which are salts thereof. When two or more substituents are present, they may be present individually or in combination of two or more kinds.

The hydrophilic substituents represented by R¹ preferably comprise a group containing at least one atom selected from the group consisting of boron, nitrogen, oxygen, phosphorus, sulfur, and halogen, from the viewpoint of improving water solubility, promoting dispersion into cell membranes and inside cells, or improving transdermal absorbability. Among them, from the viewpoint of suppressing reactions with biomolecules, the hydrophilic substituents preferably comprise one or more selected from the group consisting of tertiary amino group, quaternary ammonium group, and carbonyl group (excluding aldehyde groups) .

In Formula (1), at least one R¹ is preferably selected from a methyl group, an ethyl group, an n-propyl group, an i-propyl group, an n-butyl group, an i-butyl group, a sec-butyl group, a t-butyl group, an ethynyl group, a propargyl group, or a substituted or unsubstituted 1,2,3-triazole or 1,2,4-triazole group having a C1-4 alkyl group.

In Formula (1), R² is a substituted or unsubstituted methylene group. When a plurality of R² groups are present, they may be the same or different.

Examples of substituents of the methylene group represented by R² include carboxyl group, hydroxyl group, sulfo group, amino group, amido group, halogen atom, and groups which are salts thereof. When two or more substituents are present, they may be present individually or in combination of two or more kinds.

In Formula (1), n¹ is an integer of 0-4. When n¹ is 2 or more, the respective groups may be the same or different. A preferred example of n¹ is 1 or 2.

In Formula (1), n is an integer of 1-4. When n is 2 or more, the respective groups may be the same or different. A preferred example of n is 1 or 2. The group including R¹ may be located on any ring of the pyrene skeleton.

In Formula (1), n² is an integer of 0-4. When n² is 2 or more, the respective groups may be the same or different.

In Formula (1), R³ and R⁴ are each independently selected from hydrogen atom, a substituted or unsubstituted C1-6 alkyl group, a C2-6 alkenyl group, or a C2-6 alkynyl group, and R³ and R⁴ may together form a cyclic structure.

Examples of the C1-6 alkyl group represented by R³ and R⁴ include straight-chain or branched alkyl groups such as methyl, ethyl, n-propyl, isopropyl, n-butyl, iso-butyl, sec-butyl, tert-butyl, pentyl, isopentyl, neopentyl, and n-hexyl groups.

Examples of the C2-6 alkenyl group represented by R³ and R⁴ include straight-chain or branched alkenyl groups such as vinyl and allyl groups.

Examples of the C2-6 alkynyl group represented by R³ and R⁴ include straight-chain or branched alkynyl groups such as ethynyl and propargyl groups.

Examples of substituents of the alkyl group and the like represented by R³ and R⁴ include, independently, carboxyl group, hydroxyl group, sulfo group, amino group, amido group, halogen atom, and groups which are salts thereof. When two or more substituents are present, they may be present individually or in combination of two or more kinds.

The hydrophilic substituents represented by R³ and R⁴ preferably comprise a group containing at least one atom selected from the group consisting of boron, nitrogen, oxygen, phosphorus, sulfur, and halogen, from the viewpoint of improving water solubility, promoting dispersion into cell membranes and inside cells, or improving transdermal absorbability. Among them, from the viewpoint of suppressing reactions with biomolecules, the hydrophilic substituents preferably comprise one or more selected from the group consisting of tertiary amino group, quaternary ammonium group, and carbonyl group (excluding aldehyde groups) .

In Formula (1), R³ and R⁴ may together form a cyclic structure. When R³ and R⁴ together form a cyclic structure, examples of such cyclic structure include pyrrolidine ring, imidazolidine ring, oxazolidine ring, piperidine ring, piperazine ring, and morpholine ring structures. The cyclic structure may further have the above-described hydrophilic substituents. In addition, R³ and R⁴ may form a ring structure together with the pyrene skeleton (for example, julolidine compounds).

In Formula (1), for example, R³ and R⁴ are each independently preferably a methyl group, an ethyl group, an n-propyl group, an i-propyl group, an n-butyl group, an i-butyl group, a sec-butyl group, or a t-butyl group, which may be substituted or unsubstituted with a carboxyl group, hydroxyl group, sulfo group, amino group, amido group, halogen atom, or groups which are salts thereof.

In Formula (1), for example, at least one pair of R³ and R⁴ preferably form a pyrrolidine ring or a piperidine ring.

In Formula (1), at least one of R¹, R³, or R⁴ preferably includes at least one hydrophilic substituent selected from the group consisting of carboxyl group, hydroxyl group, sulfo group, amino group, amido group, halogen atom, and groups which are salts thereof. When two or more hydrophilic substituents are present, they may be the same or different.

In Formula (1), m is an integer of 1-4. When m is 2 or more, the respective groups may be the same or different. As a preferred example, m is 1 or 2. The group including R³ and R⁴ may be located on any ring of the pyrene skeleton.

In Formula (1), R⁵ is each independently a C1-12 alkyl group, a C2-12 alkenyl group, a C2-12 alkynyl group, a C5-12 aryl group, a C5-12 heteroaryl group, a halogen atom, or a hydrophilic substituent comprising at least one atom selected from the group consisting of boron, nitrogen, oxygen, phosphorus, sulfur, and halogen.

Examples of the C1-12 alkyl group represented by R⁵ include straight-chain or branched alkyl groups such as methyl, ethyl, n-propyl, isopropyl, n-butyl, iso-butyl, sec-butyl, tert-butyl, pentyl, isopentyl, neopentyl, n-hexyl, n-heptyl, n-octyl, n-nonyl, n-decyl, n-undecyl, and n-dodecyl groups.

Examples of the C2-12 alkenyl group represented by R⁵ include straight-chain or branched alkenyl groups such as vinyl and allyl groups.

Examples of the C2-12 alkynyl group represented by R⁵ include straight-chain or branched alkynyl groups such as ethynyl and propargyl groups.

Examples of the C5-12 aryl group represented by R⁵ include aromatic hydrocarbon groups such as phenyl, tolyl, xylyl, naphthyl, methylnaphthyl, anthracyl, and indenyl groups.

Examples of the C5-12 heteroaryl group represented by R⁵ include aromatic hydrocarbon groups in which one or more carbon atoms of the aromatic ring are substituted with atoms such as nitrogen, oxygen, or sulfur. Specific examples include pyrrolyl, pyridinyl, imidazolyl, thienyl, furanyl, pyrazolyl, oxazolyl, and thiazolyl groups.

Examples of the halogen atom represented by R⁵ include fluorine, chlorine, bromine, and iodine atoms.

The hydrophilic substituents represented by R⁵ may be appropriately the same as those described for R¹, R³, and R⁴.

As a preferred example, in Formula (1), R⁵ is each independently a methyl group, an ethyl group, a hydroxyl group, or an amino group.

In Formula (1), a is each independently an integer of 0-4. When a is 2 or more, the respective R⁵ groups may be the same or different. The R⁵ group may be located on any ring of the pyrene skeleton.

The maximum absorption wavelength of the compound is, for example, between 300 and 550 nm in 20 mM phosphate buffer (pH 7.4) at 25 °C. The maximum absorption wavelength of the compound may be within a range between any two values selected from the group consisting of 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, and 550 nm.

When the maximum fluorescence wavelength in methanol at 25 °C is defined as λMet, and the maximum fluorescence wavelength in n-heptane at 25 °C is defined as AHep, the difference between λMet and λHep of the compound is, for example, 50 nm or more, 60 nm or more, 70 nm or more, 80 nm or more, 90 nm or more, 100 nm or more, or 110 nm or more. The larger the difference between λMet and AHep, the more preferable it is, since tumor tissues can be detected in higher contrast relative to normal tissues, or cancer cells can be detected in higher contrast relative to normal cells. The difference between λMet and AHep of the compound may also be 300 nm or less, 200 nm or less, or 150 nm or less.

The maximum two-photon absorption wavelength of the compound is preferably between 600 and 1200 nm in 20 mM phosphate buffer (pH 7.4) at 25 °C. Among these, a longer wavelength than 900 nm is more preferable. Such compounds can be excited with light of a wavelength that is less absorbed by biomolecules in tissues, and are thus suitable for use in two-photon microscopy observation.

The compound also has excellent water solubility. For example, the concentration of the compound in water is preferably 1 nM to 100 mM. Depending on the intended purpose and use, the lower limit of the concentration of the compound in water may be, for example, 1 nM, 2 nM, 3 nM, 5 nM, 10 nM, 15 nM, 20 nM, 30 nM, 50 nM, 100 nM, 200 nM, 300 nM, 500 nM, 700 nM, 1 µM, 2 µM, 3 µM, 5 µM, 10 µM, 15 µM, 20 µM, 30 µM, 50 µM, 100 µM, 200 µM, 300 µM, 500 µM, 700 µM, 800 µM, 1 mM, 2 mM, 3 mM, 5 mM, 10 mM, 20 mM, 30 mM, 50 mM, 80 mM, or 100 mM. Depending on the intended purpose and use, the upper limit of the concentration of the compound in water may be, for example, 100 mM, 80 mM, 50 mM, 30 mM, 20 mM, 10 mM, 5 mM, 3 mM, 2 mM, 1 mM, 700 µM, 500 µM, 300 µM, 200 µM, 100 µM, 50 µM, 30 µM, 20 µM, 15 µM, 10 µM, 5 µM, 3 µM, 2 µM, 1 µM, 700 nM, 500 nM, 300 nM, 200 nM, 100 nM, 50 nM, 30 nM, 20 nM, or 10 nM. Any combination of the above lower and upper limits is possible. When two or more compounds are used together, the concentration of the compound refers to the total amount. For imaging of cells and/or tissues, the concentration of the compound in water is preferably within the above range.

In the case of tissue imaging such as skin, the concentration of the compound in water is preferably 100 µM to 1 mM. Depending on the intended purpose and use, the lower limit of the concentration of the compound in water may be, for example, 100 µM, 200 µM, 300 µM, 500 µM, 700 µM, or 800 µM. Depending on the intended purpose and use, the upper limit of the concentration of the compound in water may be, for example, 1 mM, 700 µM, 500 µM, 300 µM, 200 µM, or 100 µM. Any combination of the above lower and upper limits is possible. When two or more compounds are used together, the concentration of the compound refers to the total amount. For use in tissue imaging, the concentration of the compound in water is preferably within the above range.

In the case of cell imaging, the concentration of the compound in water is preferably 1 nM to 100 µM. Depending on the intended purpose and use, the lower limit of the concentration of the compound in water may be, for example, 1 nM, 2 nM, 3 nM, 5 nM, 10 nM, 15 nM, 20 nM, 30 nM, 50 nM, 100 nM, 200 nM, 300 nM, 500 nM, 700 nM, 1 µM, 2 µM, 3 µM, 5 µM, 10 µM, 15 µM, 20 µM, 30 µM, or 50 µM. Depending on the intended purpose and use, the upper limit of the concentration of the compound in water may be, for example, 100 mM, 80 mM, 50 mM, 30 mM, 20 mM, 10 mM, 5 mM, 3 mM, 2 mM, 1 mM, 700 µM, 500 µM, 300 µM, 200 µM, 100 µM, 50 µM, 30 µM, 20 µM, 15 µM, 10 µM, 5 µM, 3 µM, 2 µM, 1 µM, 700 nM, 500 nM, 300 nM, 200 nM, 100 nM, 50 nM, 30 nM, 20 nM, or 10 nM. Any combination of the above lower and upper limits is possible. When two or more compounds are used together, the concentration of the compound refers to the total amount. For use in cell imaging, the concentration of the compound in water is preferably within the above range.

The molecular weight of the above compound is, for example, 700 or less, and may be 650 or less, 600 or less, or 500 or less. The lower limit may be, for example, 100 or more or 150 or more.

### ("Salts" of the compound, etc.)

In the present invention, the term "salt of the compound" refers to a form in which a part or the entirety of the above compound is in a salt form. For example, the amino group portion of the compound may be present as an ammonium cation and form a salt with a counter anion such as a halide ion, an organic anion, or an inorganic anion. In addition, the compound of the present invention may form a salt as a zwitterion in which a cation and an anion are both present within the same molecule.

In the present invention, the above compound also includes derivatives generated by chemical modification of a small portion within the molecule, including simple substituted derivatives, adducts, solvates such as hydrates, and those referred to as analogs.

### (Method for producing the compound)

The compound of the present invention may be produced by appropriately employing known methods in addition to the methods described herein.

For example, in the case of Compound A and Compound B described below, they can be synthesized according to the following scheme.

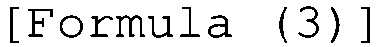

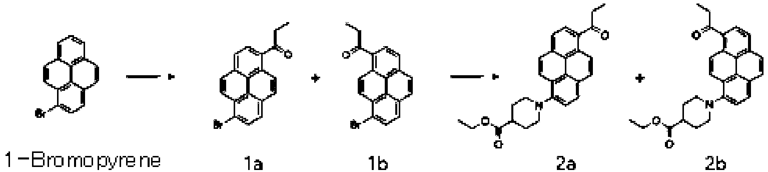

### [Formula (4)]

### [Formula (5)]

For example, the synthesis of Compound A and Compound B may be carried out by first substituting 1-bromopyrene with a propionyl group using propionyl chloride (to obtain Compound 1a and Compound 1b), then substituting the bromo group on the pyrene ring with ethyl 4-piperidinecarboxylate (to obtain Compound 2a and Compound 2b), followed by hydrolyzing the ester group of Compounds 2a and 2b under alkaline conditions.

### [Fluorescent Dye]

The fluorescent dye of the present invention comprises the above compound or a salt thereof.

By using the fluorescent dye of the present invention, cells and the like can be stained easily and rapidly.

The above fluorescent dye can be used, for example, for detecting cells (e.g., tumor cells) and/or tissues (e.g., skin tissues) derived from living organisms.

The above fluorescent dye can be used, for example, for morphological detection of cells and/or tissues. It can be suitably used, for instance, for morphological detection of tumor cells or skin cancer cells.

The above fluorescent dye can be used, for example, for staining or visualization of biological samples.

The above fluorescent dye can be used, for example, for fluorescent imaging.

The above fluorescent dye can be used, for example, for in vivo observation. The fluorescent dye is particularly suitable for in vivo observation and allows clear imaging, which has not been observed with conventional techniques, to be performed simply and rapidly.

The above fluorescent dye can be used, for example, for transdermal absorption. The fluorescent dye has high permeability into skin tissue and is particularly suitable for systems in which observation is conducted via transdermal absorption, allowing clear imaging, which has not been observed with conventional techniques, to be performed simply and rapidly.

The tissues and cells to which the fluorescent dye of the present invention is applicable are not particularly limited, and may be derived from animals or plants, live cells or dead cells, naturally derived, artificially synthesized, or cultured.

The living organisms are not particularly limited as long as they are multicellular animals, but are preferably mammals, and more preferably humans.

Examples of tissues include skin, brain, spinal cord, esophagus, stomach, small intestine, large intestine, duodenum, rectum, liver, pancreas, gallbladder, bladder, kidney, heart, spleen, thymus, prostate, uterus, ovary, testis, mammary gland, lung, bronchus, eyeball, nose, paranasal sinus, oral cavity, pharynx, salivary gland, thyroid, parathyroid, adrenal gland, muscle, bone marrow, blood vessel, nerve, lymph node, peritoneum, diaphragm, and blood. In one embodiment, the tissue to which the fluorescent dye of the present invention is applied is skin, such as epidermis, dermis, or a combination thereof.

The fluorescent dye of the present invention can be applied to tissues separated from a living body by surgical procedures such as excision, resection, puncture, or blood sampling, or tissues obtained from excreta such as feces, urine, or sweat, or other body fluids.

In the present specification, the term "tissues derived from a living body" encompasses not only tissues separated from a living body, but also the living body itself or tissues that are part of the living body and not separated from it.

In one embodiment, the morphology of the tissue may be appropriately selected depending on the detection method, and may be, for example, an organ itself, a section thereof, or a three-dimensional fragment thereof.

Depending on the morphology, the tissue may have been subjected to treatments such as fixation with formalin, paraffin embedding, deparaffinization, dehydration, or clearing.

In one embodiment, the tumor cells are cells derived from skin tumors. Such tumor cells include, for example, sweat gland tumors (extramammary Paget's disease, mammary Paget's disease, eccrine porocarcinoma, microcystic adnexal carcinoma, cutaneous mucinous carcinoma, etc.), malignant melanoma, epidermal/hair follicle tumors (basal cell carcinoma, squamous cell carcinoma, actinic keratosis, Bowen's disease, leukoplakia, keratoacanthoma, etc.), neural tumors (Merkel cell carcinoma, malignant peripheral nerve sheath tumor, etc.), and mesenchymal tumors (dermatofibrosarcoma protuberans, solitary fibrous tumor, muscle-derived tumors, liposarcoma, angiosarcoma, Kaposi's sarcoma, spindle cell hemangioendothelioma, atypical fibroxanthoma, epithelioid sarcoma, synovial sarcoma, undifferentiated pleomorphic sarcoma, etc.).

In the fluorescent dye of the present invention, the compound is not particularly limited as long as it can be applied to tissues and the like.

In one embodiment, the compound may distribute into cell membranes, the inside of cells, and secretions, and in a further embodiment, the compound may insert into cell membranes.

Examples of such secretions include bile, hormones, digestive enzymes, exosomes, amyloid, and insulin.

In one embodiment, the compound exhibits strong fluorescence anisotropy in the cell membrane.

In one embodiment, the compound exhibits lower fluorescence intensity outside cells than in cell membranes and/or inside cells. In a specific embodiment, substantially no fluorescence is observed outside the cells. Although not limited thereto, for example, the fluorescence intensity outside the cells may be 50% or less, 40% or less, 30% or less, 20% or less, 10% or less, 5% or less, or 1% or less relative to the fluorescence intensity inside the cells. Comparison of fluorescence intensities inside and outside cells is performed by exciting cells derived from tissues of interest with light of a wavelength capable of exciting the compound, acquiring fluorescence microscopy images under conditions capable of detecting light at the maximum fluorescence wavelength, and comparing the average fluorescence signal intensities inside and outside the cells.

The fluorescent dye of the present invention may be used alone or as a mixture of two or more types.

The fluorescent dye of the present invention may also be used in combination with other components known to be used in fluorescent dyes, in addition to the above compound or salts thereof.

Examples of such other components include one or more selected from pH buffers, surfactants, salts, solvents, and dye compositions other than Compound (1).

Examples of pH buffers include one or more selected from tris(hydroxymethyl)aminomethane; Good's buffers (HEPES, MOPS, etc.); and pH buffers containing citric acid, acetic acid, lactic acid, oxalic acid, phthalic acid, imidazole, triethanolamine, diethanolamine, glycine, boric acid, phosphoric acid, or carbonic acid.

Examples of solvents include one or more selected from water, ethanol, methanol, 2-propanol, dimethyl sulfoxide, N,N-dimethylformamide, acetonitrile, acetone, ethyl acetate, tetrahydrofuran, 1,2-dichloroethane, and phosphate buffer. Among these, water, aqueous solutions, and phosphate buffer are preferable.

When the fluorescent dye is prepared as a solution, the concentration of Compound (1) is preferably 1 µM to 1 mM relative to the total fluorescent dye, and may be, depending on the intended purpose and use, 2 µM to 800 µM, 3 µM to 700 µM, 5 µM to 600 µM, 10 µM to 500 µM, 30 µM to 450 µM, 50 µM to 400 µM, 100 µM to 350 µM, or 200 µM to 300 µM. When two or more Compounds (1) are used together, the concentration refers to the total amount thereof. By virtue of high water solubility, the fluorescent dye enables clear imaging, not observed with conventional techniques, to be performed simply and rapidly.

When the fluorescent dye is in a solid form, the amount of Compound (1) is preferably 0.1 mass% to 100 mass% relative to the total fluorescent dye, and may be, depending on the intended purpose and use, 1 mass% to 95 mass%, 3 mass% to 90 mass%, 5 mass% to 85 mass%, 10 mass% to 80 mass%, 15 mass% to 75 mass%, 20 mass% to 70 mass%, 30 mass% to 60 mass%, or 40 mass% to 50 mass%. When two or more Compounds (1) are used together, the amount refers to the total amount thereof. By virtue of high water solubility, the fluorescent dye enables clear imaging, not observed with conventional techniques, to be performed simply and rapidly.

The dye composition other than Compound (1) is not particularly limited as long as it does not interfere with the detection of cells by Compound (1). Examples include nuclear staining dyes such as propidium iodide (PI), ethidium bromide, acridine orange, DAPI, and Hoechst, which do not substantially interfere with staining by the compound and can be suitably used. In addition, examples of dye compositions other than Compound (1) include those used for various tissue staining, such as hematoxylin-eosin (HE) staining, Azan staining, Masson's trichrome staining, Elastica-Van Gieson staining, silver impregnation staining, Victoria blue staining, PAM staining, PTAH staining, Sudan III staining, Oil Red O staining, PAS staining, Alcian blue staining, toluidine blue staining, colloidal iron staining, mucicarmine staining, Congo red staining, dyllon staining, Grimelius staining, Fontana-Masson staining, Kossa staining, Prussian blue staining, Bodian staining, Klüver-Barrera staining, and Giemsa staining. These dye compositions may be used singly or in combination of two or more kinds.

The fluorescent dye of the present invention may be in solid form such as powder, or in liquid form.

When tissues are stained with the fluorescent dye of the present invention, Compound (1) may distribute into each portion of the intracellular lipid membrane. Depending on the phase (composition) of the lipid membrane, the fluorescence wavelength shifts, resulting in a change in fluorescence color. Therefore, by appropriately selecting the fluorescence wavelength to be detected, changes in composition or phase state of the intracellular lipid membrane can be detected with high contrast.

When tissues are stained with the fluorescent dye of the present invention, Compound (1) may distribute into both tumor cells and normal tissue cells; however, the fluorescence wavelength of Compound (1) in tumor cells shifts from that in normal tissue cells. Therefore, by appropriately selecting the fluorescence wavelength to be detected, tumor cells can be detected in high contrast relative to normal tissue. Thus, the fluorescent dye of the present invention can be used for examination or diagnosis of tumors, particularly malignant tumors.

In addition, when applied to a living body, the fluorescent dye of the present invention can be used, for example, for diagnosis of tumors, particularly malignant tumors. Furthermore, the fluorescent dye of the present invention may be applied to a living body before, during, or after a surgical procedure, in order to identify the resection margin of a tumor, particularly cancer, or to confirm whether any residual tumor remains after resection.

The fluorescent dye of the present invention may be a reagent used in clinical tests or basic research, and may also be a pharmaceutical or quasi-drug.

### [Staining Material]

The staining material of the present invention is for staining lipid bilayers, oil droplets, or protein aggregates present in cells or modeling a cellular environment, and comprises the above compound or a salt thereof.

The staining material of the present invention can suitably stain protein aggregates such as amyloids.

The staining material may appropriately incorporate the matters described above with respect to Compound (1) or the fluorescent dye.

For components other than the above compound or salts thereof, the staining material may employ new or known means as appropriate depending on the intended purpose or use.

### [Kit]

The kit of the present invention comprises the above compound or a salt thereof.

By using the kit of the present invention, cells and the like can be stained easily and rapidly.

The configuration included in the kit may appropriately adopt the matters described above in the section on the fluorescent dye.

The kit may also be prepared by combining the above compound or a salt thereof with reagents or instruments for staining or preparation of tissue specimens.

In one embodiment, the kit includes reagents for preparing a staining solution. In a more specific embodiment, the reagents for preparing the staining solution may comprise one or more selected from the group consisting of the above-described pH buffers, surfactants, salts, solvents, and other dye compositions.

### [Method for Detecting Cells]

The method for detecting cells of the present invention comprises step (1) of staining cells with a fluorescent dye containing the above compound or a salt thereof.

By using the method for detecting cells of the present invention, cells and the like can be detected easily.

The fluorescent dye used in step (1) may appropriately adopt the matters described above regarding the fluorescent dye.

The descriptions of the respective components in the above section on the fluorescent dye may be appropriately adopted as well.

The step of staining cells in step (1) may employ materials and methods used in known cell staining processes.

The method for detecting cells of the present invention may further comprise step (2) of evaluating by using fluorescence spectra measured in two or more solvents after step (1).

Since the fluorescent dye of the present invention may be a compound exhibiting solvatochromism, the absorption maximum wavelength and/or fluorescence maximum wavelength of the compound or fluorescent dye changes depending on the polarity (hydrophobicity) of its environment. Therefore, in step (2), by using such a fluorescent dye, the evaluation can be easily carried out, for example, morphological detection of cells and/or tissues or staining and visualization of biological samples, by using fluorescence spectra measured in two or more solvents.

The step of evaluation using fluorescence spectra in step (2) may employ known techniques for measuring fluorescence spectra of compounds, tissues, or other materials.

Examples of solvents that may be used in step (2) include one or more selected from water, ethanol, methanol, 2-propanol, dimethyl sulfoxide, N,N-dimethylformamide, acetonitrile, acetone, ethyl acetate, tetrahydrofuran, and 1,2-dichloroethane.

Step (2) is preferably carried out by using fluorescence imaging. For example, two-photon fluorescence imaging can also be suitably employed.

In one embodiment, the method of the present invention is applied to tissues such as an organ itself or a three-dimensional fragment thereof. In such an embodiment, the method preferably includes a step of clearing the tissue prior to staining. Examples of tissue clearing methods include TDE method, LUCID method, CLARITY method, PACT/PARS method, CUBIC method, 3DISCO method, Scale method, ScaleS method, SeeDB method, FocusClear method, Clear method, BABB method, iDISCO method, and uDISCO method. These clearing methods are described, for example, in Cell Chem. Biol., 2016, Vol. 23, pp. 137-157, and Laser Photonics Rev., 2019, Vol. 13, 1800292.

In one embodiment, the method of the present invention is applied to a living body itself or a tissue that is part of the living body and not separated from it.

In one embodiment, the method of the present invention is applied to tissue sections. The tissue sections may optionally undergo treatments such as fixation (e.g., with formalin), dehydration, de-alcoholization, paraffin infiltration, paraffin embedding, deparaffinization, rehydration, or staining using the above-described tissue staining methods.

The staining is generally carried out by contacting the tissue with a staining solution containing the above compound. The concentration of the compound in the staining solution relative to the total amount of the staining solution is adjusted, for example, to 0.001 mg/mL or more, 0.01 mg/mL or more, 0.1 mg/mL or more, 0.2 mg/mL or more, 0.3 mg/mL or more, 0.4 mg/mL or more, 0.5 mg/mL or more, 0.6 mg/mL or more, 0.7 mg/mL or more, 0.8 mg/mL or more, 0.9 mg/mL or more, or 1 mg/mL or more.

The concentration of the compound in the staining solution relative to the total amount of the staining solution may be adjusted, for example, to 500 mg/mL or less, 200 mg/mL or less, 100 mg/mL or less, 50 mg/mL or less, 20 mg/mL or less, 10 mg/mL or less, 5 mg/mL or less, or 2 mg/mL or less.

The temperature during staining is not particularly limited, but is, for example, 0-80 °C, 4-50 °C, 20-45 °C, or 25-40 °C, and is preferably 35-42 °C.

The time for contacting the tissue with the staining solution is, for example, 1 minute or more, 10 minutes or more, 20 minutes or more, 1 hour or more, 2 hours or more, or 1 day or more, and, for example, 14 days or less or 7 days or less.

In one embodiment, the time for contacting the tissue with the staining solution is, at 0-40 °C, 12 hours or less, preferably 6 hours or less, more preferably 2 hours or less, even more preferably 1 hour or less, and still more preferably 30 minutes or less, and most preferably 10 minutes or less. The time may also be, for example, 1 minute or more, 2 minutes or more, 5 minutes or more, or 10 minutes or more.

Tissues and the like stained with a staining solution containing the above compound can be used as such for morphological detection of cells and/or tissues and for detection of tumor cells. Optionally, prior to detection, the tissues may be subjected to additional treatments such as staining with another dye composition.

The method of the present invention may further include a step of detecting tumor cells. Detection of tumor cells can be carried out, for example, by exciting the above compound with light of an appropriate wavelength and detecting the emitted fluorescence. For detection, a confocal laser scanning microscope can be used; depending on section thickness, instruments capable of multiphoton excitation, such as a two-photon microscope, may be employed. For example, when 1-acetyl-6-piperidylpyrene (PK) is used as the compound, it is suitable for measurement by two-photon microscopy as shown in the Examples.

In one embodiment, cells such as tumor cells are detected by selecting fluorescence that includes a single specific wavelength at which contrast is obtained between the tumor cells and normal tissue cells, and measuring the fluorescence intensity.

In another embodiment, detection of cells such as tumor cells is performed by multi-wavelength measurement; that is, the cells are detected by detecting fluorescence including two or more different specific wavelengths and integrating the respective fluorescence intensities.

In one embodiment, when Compound A in the Examples is used as the compound, the fluorescence to be detected includes one or two or more wavelengths selected from the ranges of 350-750 nm, 550-700 nm, 600-700 nm, and 650-750 nm.

By the detection method of the present invention, examinations and diagnoses of cells such as tumors, and identification of tumor resection margins can be performed, as described in the section on the fluorescent dye above.

In addition to merging, ratiometric analysis-which detects fluorescence at two different wavelengths and calculates the ratio of their fluorescence intensities-is also effective as an image analysis method utilizing solvatochromism.

Besides fluorescence imaging, fluorescence lifetime imaging can also be used as appropriate.

### Examples

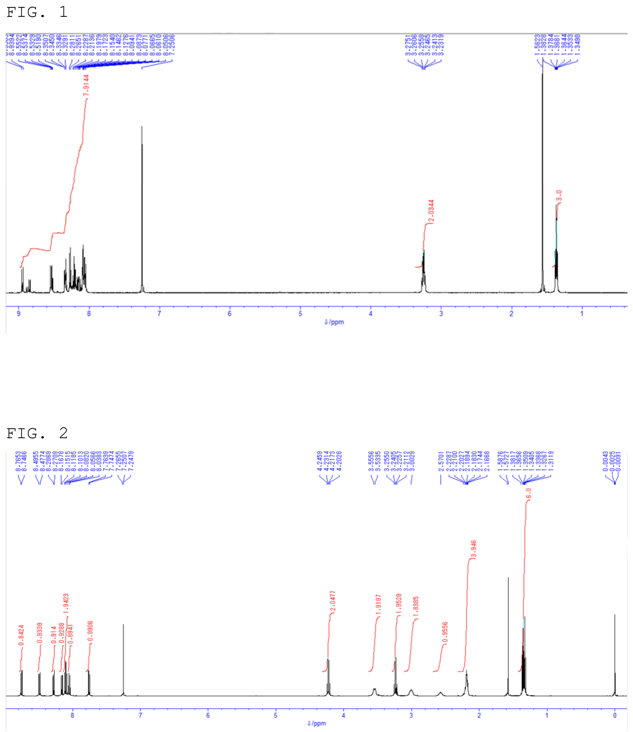

Next, the present invention will be described in more detail with reference to the following Examples. However, the invention is not limited to these Examples.
(¹H-NMR Analysis)
¹H-NMR analyses in the Examples were performed using a nuclear magnetic resonance spectrometer (manufactured by JEOL Ltd.: JNM-ECA 500). The results obtained for each compound are shown in Figures 1-4 and 7-13.

### (Synthesis of Compounds A and B)

The synthesis of Compounds A and B was carried out according to the scheme below.

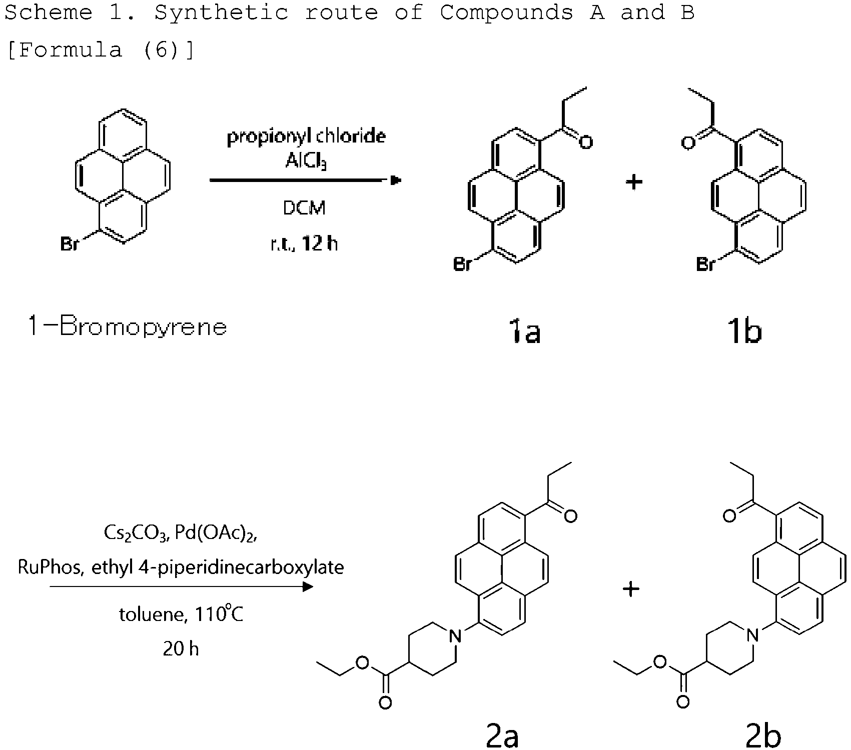

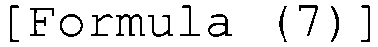

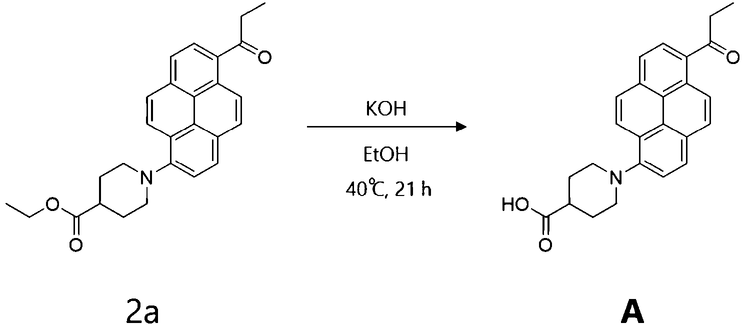

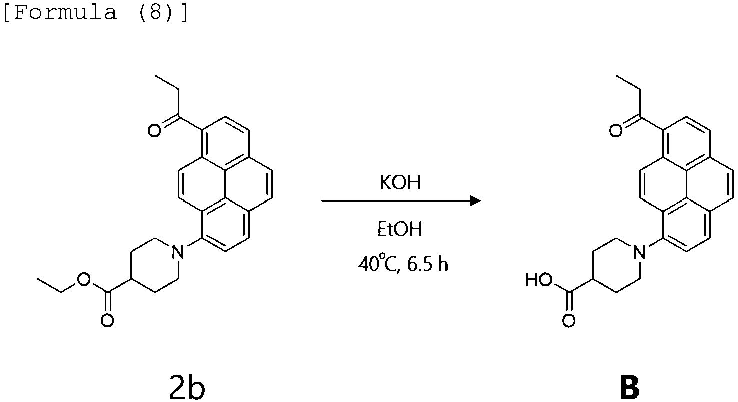

### Example 1

### (Synthesis of Compounds 1a and 1b)

Into a two-necked round-bottom flask were added 1-bromopyrene (2.00 g, 7.11 mmol), and the flask was purged with argon. Dehydrated dichloromethane (36 mL) was added, and the mixture was cooled in an ice bath. To this solution was added aluminum chloride (1.04 g, 7.82 mmol) and stirred for 15 minutes, followed by dropwise addition of propionyl chloride (0.744 mL, 8.53 mmol). The mixture was stirred at room temperature for 12 hours. The reaction was quenched by addition of water, and the mixture was extracted with dichloromethane. The organic layer obtained was washed with saturated brine and dried over magnesium sulfate. After concentration under reduced pressure, the residue was purified by medium-pressure silica gel column chromatography (Hexane:DCM = 75:25 → 70:30). As a result, a mixture of the desired Compounds 1a and 1b was obtained as a yellow solid (1.64 g, 68%, Figure 1).
· ¹H NMR (500 MHz, CDCl₃, TMS): δ 8.96-8.05 (m, 8H), 3.27-3.22 (m, 2H), 1.38-1.34 (m, 3H) ppm.

### Example 2

### (Synthesis of Compounds 2a and 2b)

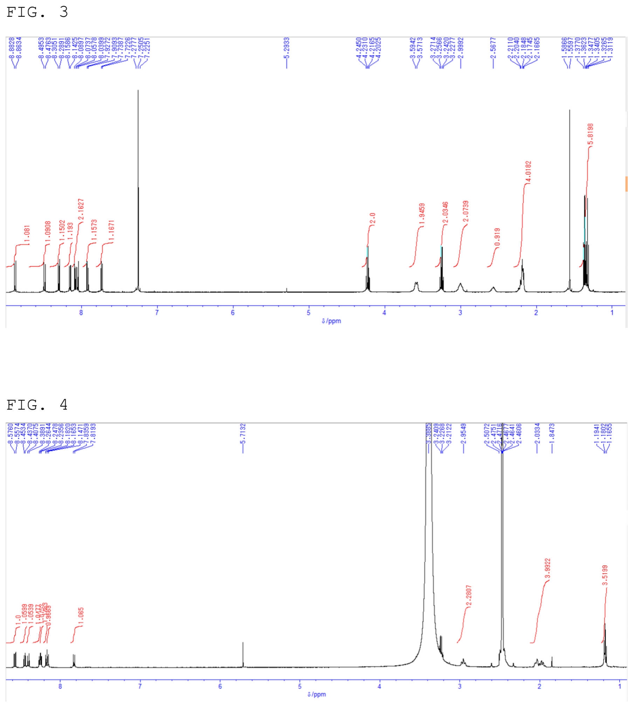

A two-necked round-bottom flask was charged with a mixture of Compounds 1a and 1b (1.53 g, 4.54 mmol), cesium carbonate (4.40 g, 13.6 mmol), palladium(II) acetate (101 mg, 0.450 mmol), RuPhos (424 mg, 0.910 mmol), and a stirring bar. After attaching a reflux condenser, the flask was purged with argon. Ethyl 4-piperidinecarboxylate (1.05 mL, 6.81 mmol) and argon-bubbled toluene (23 mL) were added, and the mixture was heated and stirred in an oil bath at 100°C for 20 hours. Water was added to the reaction solution, and the mixture was extracted with dichloromethane. The organic layer obtained was washed with saturated brine and dried over magnesium sulfate. After concentration under reduced pressure, the residue was purified by silica gel column chromatography (DCM:Hex = 2:8). As a result, the desired Compounds 2a and 2b were obtained as yellow solids (2a: 164.2 mg, 17%; 2b: 214.9 mg, 23%, Figures 2 and 3).
· 2a: ¹H NMR (500 MHz, CDCl₃, TMS): δ 8.75 (d, J = 9.5 Hz, 1H), 8.48 (d, J = 9.1 Hz, 1H), 8.28 (d, J = 8.1 Hz, 1H), 8.16 (d, J = 8.1 Hz, 1H), 8.10 (dd, J = 8.4, 9.5 Hz, 2H), 8.04 (d, J = 9.1 Hz, 1H), 7.75 (d, J = 8.4 Hz, 1H), 4.23 (q, J = 7.2 Hz, 2H), 3.56-3.53 (m, 2H), 3.25 (q, J = 7.2 Hz, 2H), 3.00 (m, 2H), 2.57 (m, 1H), 2.23-2.17 (m, 4H), 1.38-1.31 (m, 6H) ppm.
· 2b: ¹H NMR (500 MHz, CDCl₃, TMS): δ 8.87 (d, J = 8.1 Hz, 1H), 8.48 (d, J = 8.9 Hz, 1H), 8.29 (d, J = 8.9 Hz, 1H), 8.15 (d, J = 8.0 Hz, 1H), 8.06 (d, J = 8.1 Hz, 1H), 8.04 (d, J = 8.0 Hz, 1H), 7.91 (d, J = 9.6 Hz, 1H), 7.73 (d, J = 9.6 Hz, 1H), 4.23 (q, J = 7.2 Hz, 2H), 3.59-3.57 (m, 2H), 3.25 (q, J = 7.2 Hz, 2H), 3.00 (m, 2H), 2.57 (m, 1H), 2.21-2.17 (m, 4H), 1.38-1.31 (m, 6H) ppm.

### Example 3

### (Synthesis of Compound A)

Compound 2a (53.3 mg, 0.129 mmol) was placed in a round-bottom flask. To this was added 0.25 M ethanolic potassium hydroxide solution (2.58 mL), and the mixture was stirred in an oil bath at 40°C for 21 hours. The reaction was quenched by dropwise addition of dilute hydrochloric acid, and the mixture was extracted with dichloromethane. The organic layer obtained was washed with saturated brine and dried over magnesium sulfate. After concentration under reduced pressure, the residue was purified by medium-pressure silica gel chromatography (DCM → DCM:MeOH = 88:12). As a result, the desired Compound A was obtained as a yellow solid (25.2 mg, 58%, Figure 4).
· A: ¹H NMR (500 MHz, DMSO-d₆): δ 8.57 (d, J = 9.3 Hz, 1H), 8.45 (d, J = 8.1 Hz, 1H), 8.40 (d, J = 9.3 Hz, 1H), 8.26 (d, J = 8.2 Hz, 1H), 8.25 (d, J = 8.2 Hz, 1H), 8.18 (d, J = 7.9 Hz, 1H), 8.16 (d, J = 7.9 Hz, 1H), 7.83 (d, J = 8.1 Hz, 1H), 2.95 (m, 2H), 2.10-1.90 (m, 4H), 1.19 (t, J = 7.2 Hz, 3H) ppm. (Signals derived from -N-CH₂-, -COCH₂-, and -CHCO-were buried under solvent and water peaks.)

### Example 4

### (Synthesis of Compound B)

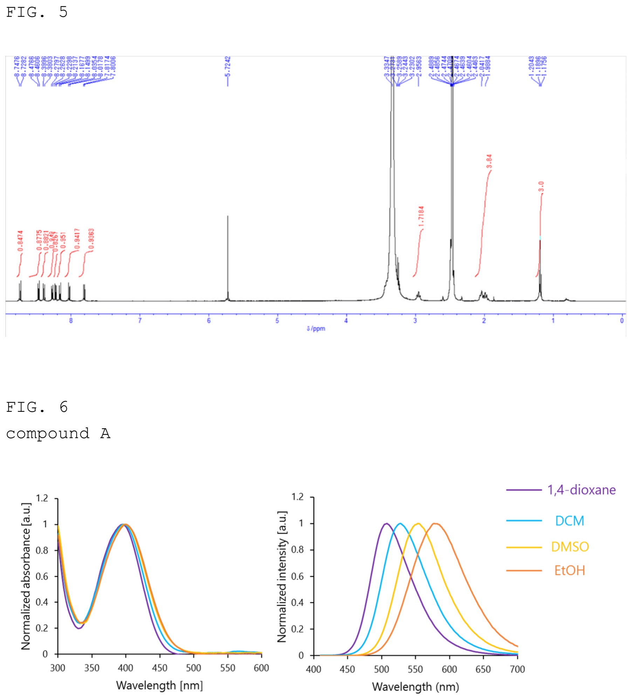

Compound 2b (123 mg, 0.297 mmol) was placed in a round-bottom flask. To this was added 0.25 M ethanolic potassium hydroxide solution (5.95 mL), and the mixture was stirred in an oil bath at 40°C for 6.5 hours. The reaction was quenched by dropwise addition of dilute hydrochloric acid, and the mixture was extracted with dichloromethane. The organic layer obtained was washed with saturated brine and dried over magnesium sulfate. After concentration under reduced pressure, the residue was purified by medium-pressure silica gel chromatography (DCM → DCM:MeOH = 98:2). As a result, the desired Compound B was obtained as a yellow solid (98.1 mg, 85%, Figure 5).
· B: ¹H NMR (500 MHz, DMSO-d₆): δ 8.74 (d, J = 8.3 Hz, 1H), 8.47 (d, J = 9.0 Hz, 1H), 8.39 (d, J = 9.0 Hz, 1H), 8.27 (d, J = 8.1 Hz, 1H), 8.22 (d, J = 8.3 Hz, 1H), 8.16 (d, J = 9.7 Hz, 1H), 8.03 (d, J = 8.1 Hz, 1H), 7.81 (d, J = 9.7 Hz, 1H), 2.96 (m, 2H), 2.04-1.99 (m, 4H), 1.19 (t, J = 7.2 Hz, 3H) ppm. (Signals derived from -N-CH₂-, -COCH₂-, and -CHCO-were buried under solvent and water peaks.)

### Example 5

### (Photophysical measurements of Compounds A and B in solution)

The UV-visible absorption spectra and fluorescence spectra of Compounds A and B obtained in Examples 3 and 4 were measured in solution.

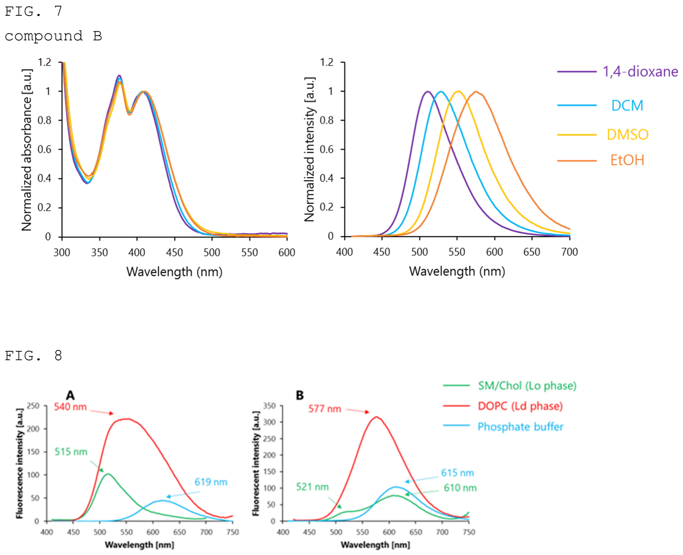

UV-visible absorption spectra were measured using a spectrophotometer (JASCO Corporation, V-670). Fluorescence spectra were measured using a spectrofluorometer (JASCO Corporation, FP-6600). The concentration of each compound in each solvent was 5 µM. The results obtained are shown in Figures 6 and 7 and in Tables 1 and 2.

**[Table 1]**

| Table 1. Photophysical parameters of Compound A in organic solvents | | | |
|---|---|---|---|
| | Absorption maximum wavelength [nm] | Fluorescence maximum wavelength [nm] | Fluorescence quantum yield |
| 1,4-dioxane | 395 | 507 | 91 |
| DCM | 397 | 526 | 90 |
| DMSO | 398 | 553 | 74 |
| EtOH | 394 | 574 | 81 |

**[Table 2]**

| **Table 2. Photophysical parameters of Compound B in organic solvents** | | | |
|---|---|---|---|
| | Absorption maximum wavelength [nm] | Fluorescence maximum wavelength nm] | Fluorescence quantum yield |
| 1.4-dioxane | 406 | 510 | 82 |
| DCM | 407 | 527 | 86 |
| DMSO | 408 | 549 | 73 |
| EtOH | 405 | 568 | 79 |

### Example 6

### (Fluorescence behavior of Compounds A and B in the presence of artificial lipid bilayers)

Two types of liposomes were prepared: one forming an ordered phase (Lo phase, lipid: sphingomyelin-cholesterol (SM/Chol)) and one forming a disordered phase (Ld phase, lipid: dioleoylphosphatidylcholine (DOPC)). To the phosphate buffer in which the liposomes were dispersed, the dye (Compound A or Compound B obtained in Examples 3 and 4) was added at a lipid:dye molar ratio of 100:1, and fluorescence spectra were measured. The results obtained are shown in Figure 8.

### Example 7

### (Transdermal staining and fluorescence imaging of live mouse skin tissue using Compounds A and B)

Transdermal staining and fluorescence imaging of live mouse skin tissue were performed using Compounds A and B obtained in Examples 3 and 4. More specifically, multiphoton fluorescence imaging (spinous layer, basal layer to dermis region; detection: <492 nm, cyan (second harmonic); 500-550 nm, green; 560-593 nm, orange; >593 nm, red; Figure 9) was performed on live mouse skin tissue (foot) stained with Compound A or Compound B (concentration 100 µM, excitation wavelength: 960 nm).

A multiphoton microscope A1R MP+ (NIKON) was used for microscopic observation. For Compound A, a laser light source at 960 nm was used for excitation. Scanning was performed to a sample depth of 200 µm. Three-dimensional reconstructed images were obtained by processing the acquired data with the accompanying software.

The mouse individuals were anesthetized, and sufficient staining was achieved by immersing the hind paw in the above staining solution at room temperature for 2-3 hours or by intravenous administration.

The results obtained are shown in Figure 9.

As shown in Figure 9, Compound A strongly stained the membranes of each cell constituting mouse skin tissue, allowing clear delineation of individual cell boundaries and visualization of tissue structures. More specifically, Compound A was particularly excellent in visualizing, for example, stratum corneum structures, stratum lucidum, intercellular bridges of keratinocytes in the spinous layer, acrosyringium structures, eccrine glands, peripheral nerves, myelin sheaths, immune cells, and migrating cells.

### (Synthesis of Compounds C-E)

The synthesis of Compounds C-E was carried out.

### [Formula (9)]

### Example 8

### (Synthesis of Compound C)

### Compound C was synthesized according to the scheme below. Scheme 2. Synthetic route of Compound C

### [Formula (10)]

According to the above scheme, synthesis reactions were carried out following the procedures of Examples 1-4 as appropriate. As a result, the desired Compound C was obtained as a yellow solid (overall yield from starting materials: 0.81%; yield of the final step: 33%, Figure 10).
· C: ¹H NMR (500 MHz, DMSO-d₆): δ 8.48 (d, J = 9.3 Hz, 1H), 8.34 (d, J = 9.3 Hz, 1H), 8.28 (d, J = 7.5 Hz, 1H), 8.25 (d, J = 7.5 Hz, 1H), 8.23 (d, J = 5.3 Hz, 1H), 8.10 (s, 1H), 8.09 (d, J = 6.8 Hz, 1H), 8.04 (t, J = 7.5 Hz, 1H), 2.95 (m, 2H), 2.05-1.99 (m, 4H), 1.19 (t, J = 7.0 Hz, 3H) ppm. (Signals derived from -N-CH₂-, -COCH₂-, and -CHCO-were buried under solvent and water peaks.)

### (Synthesis of Compounds D and E)

### Compounds D and E were synthesized according to the scheme below.

### Scheme 3. Synthetic route of Compounds D and E

### [Formula (11)]

### [Formula (12)]

### [Formula (13)]

### Example 9

### (Synthesis of Compound D)

According to the above scheme, synthesis reactions were carried out following the procedures of Examples 1-4 as appropriate. As a result, the desired Compound D was obtained as a yellow solid (overall yield from starting materials: 10%; yield of the final step: 99%, Figure 11).
· D: ¹H NMR (500 MHz, DMSO-d₆): δ 8.58 (d, J = 9.3 Hz, 1H), 8.47 (d, J = 8.1 Hz, 1H), 8.41 (d, J = 9.2 Hz, 1H), 8.26 (d, J = 8.2 Hz, 1H), 8.25 (d, J = 8.2 Hz, 1H), 8.17 (d, J = 9.2 Hz, 1H), 8.15 (d, J = 9.3 Hz, 1H), 7.82 (d, J = 8.1 Hz, 1H), 3.45 (t, J = 6.2 Hz, 2H), 3.15 (m, 4H), 2.70 (t, J = 6.2 Hz, 2H), 1.85 (m, 4H), 1.64 (m, 2H) ppm.

### Example 10

### (Synthesis of Compound E)

According to the above scheme, synthesis reactions were carried out following the procedures of Examples 1-4 as appropriate. As a result, the desired Compound E was obtained as a yellow solid (overall yield from starting materials: 5.8%; yield of the final step: 76%, Figure 12).
· E: ¹H NMR (500 MHz, DMSO-d₆): δ 8.75 (d, J = 9.6 Hz, 1H), 8.48 (d, J = 8.3 Hz, 1H), 8.40 (d, J = 9.6 Hz, 1H), 8.26 (d, J = 8.2 Hz, 1H), 8.22 (d, J = 8.2 Hz, 1H), 8.15 (d, J = 9.0 Hz, 1H), 8.01 (d, J = 9.0 Hz, 1H), 7.79 (d, J = 8.3 Hz, 1H), 3.45 (t, J = 6.2 Hz, 2H), 3.15 (m, 4H), 2.70 (t, J = 6.2 Hz, 2H), 1.85 (m, 4H), 1.64 (m, 2H) ppm.

### Example 11

### (Photophysical measurements of Compounds C-E in solution)

The UV-visible absorption spectra and fluorescence spectra of Compounds C-E obtained in Examples 8-10 were measured in solution.

UV-visible absorption spectra were measured using a spectrophotometer (JASCO Corporation, V-670). Fluorescence spectra were measured using a spectrofluorometer (JASCO Corporation, FP-6600). The concentration of each compound in each solvent was 5 µM. The results obtained are shown in Figures 13-15 and in Tables 3-5.

**[Table 3]**

| **Table 3. Photophysical parameters of Compound C in organic solvents** | | | |
|---|---|---|---|
| | Absorption maximum wavelength [nm] | Fluorescence maximum wavelength [nm] | Fluorescence quantum yield |
| 1.4-dioxane | 400 | 497 | 81 |
| DCM | 401 | 507 | 83 |
| DMSO | 401 | 530 | 40 |
| EtOH | 400 | 553 | 60 |

**[Table 4]**

| **Table 4. Photophysical parameters of Compound D in organic solvents** | | | |
|---|---|---|---|
| | Absorption maximum wavelength [nm] | Fluorescence maximum wavelength [nm] | Fluorescence quantum yield |
| 1.4-dioxane | 399 | 516 | 89 |
| DCM | 405 | 540 | 89 |
| DMSO | 401 | 562 | 88 |
| EtOH | 396 | 577 | 86 |

**[Table 5]**

| **Table 5. Photophysical parameters of Compound E in organic solvents** | | | |
|---|---|---|---|
| | Absorption maximum [nm] wavelength | Fluorescence [nm] maximum wavelength | Fluorescence quantum yield |
| 1.4-dioxane | 410 | 519 | 86 |
| DCM | 415 | 540 | 86 |
| DMSO | 414 | 559 | 89 |
| EtOH | 409 | 575 | 81 |

### (Synthesis of Compounds F-H)

The synthesis of Compounds F-H was carried out.

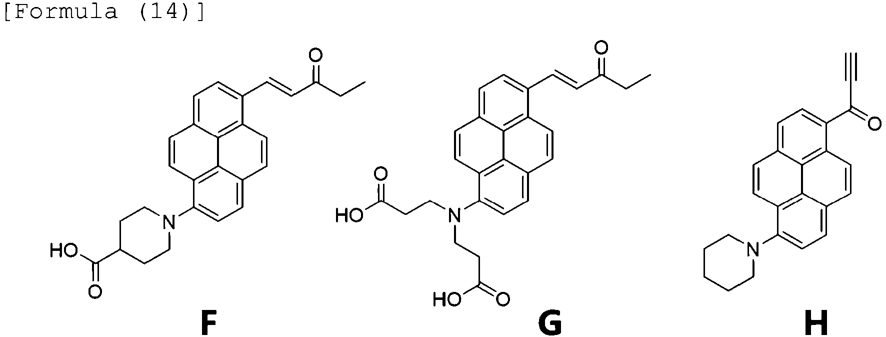

### Example 12

### (Synthesis of Compound F)

Compound F was synthesized according to the scheme below.

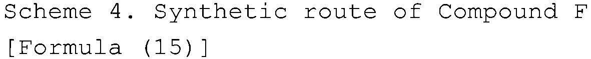

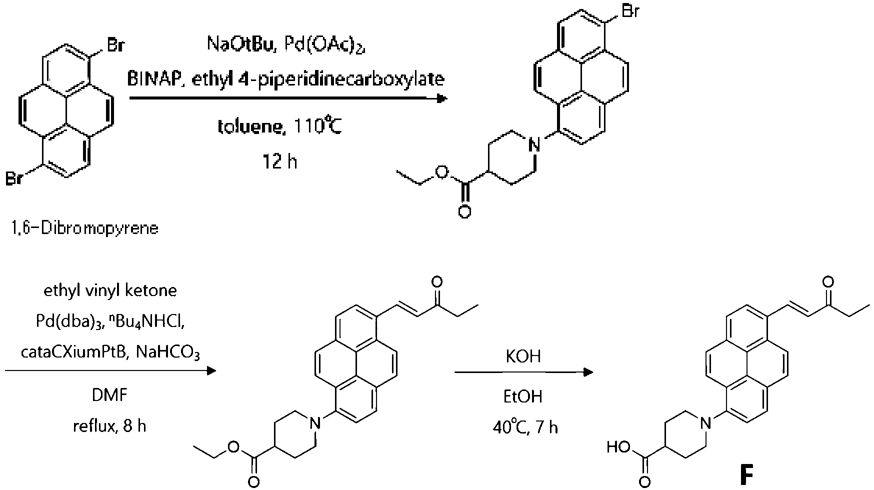

According to the above scheme, synthesis reactions were performed following Examples 1-4 as appropriate. As a result, the desired Compound F was obtained as an orange solid (overall yield from starting materials: 0.63%; yield of the final step: 11%; Figure 16).
· F: ¹H NMR (500 MHz, DMSO-d₆): δ 8.63 (d, J = 16 Hz, 1H), 8.45-8.43 (m, 2H), 8.36 (d, J = 9.1 Hz, 1H), 8.27-8.12 (m, 4H), 7.81 (d, J = 8.2 Hz, 1H), 7.11 (d, J = 16 Hz, 1H), 4.0-4.2 (m, 1H), 3.39 (m, 2H), 2.95 (t, J = 10 Hz, 2H), 2.89 (q, J = 7.2 Hz, 2H), 2.03-1.98 (m, 4H), 1.08 (t, J = 7.2 Hz, 3H) ppm.

### Example 13

### (Synthesis of Compound G)

Compound G was synthesized according to the scheme below.

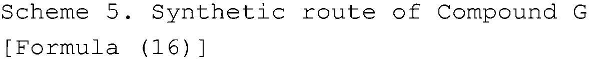

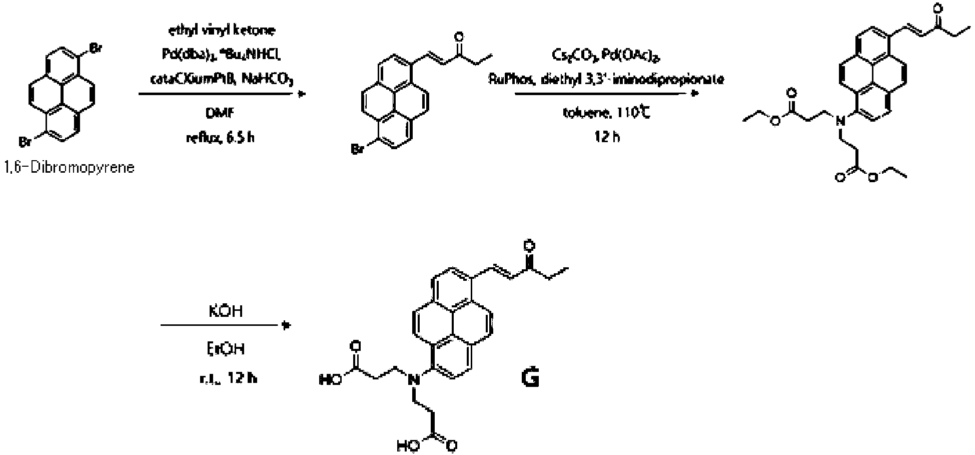

According to the above scheme, synthesis reactions were performed following Examples 1-4 as appropriate. As a result, the desired Compound G was obtained as an orange solid (overall yield from starting materials: 0.040%; yield of the final step: 8%; Figure 17).
· G: ¹H NMR (500 MHz, DMSO-d₆): δ 8.65 (d, J = 16 Hz, 1H), 8.51 (d, J = 9.2 Hz, 1H), 8.47 (d, J = 8.0 Hz, 1H), 8.42 (d, J = 9.4 Hz, 1H), 8.30 (d, J = 8.4 Hz, 1H), 8.24-8.22 (m, 2H), 8.14 (d, J = 9.2 Hz, 1H), 7.97 (d, J = 8.4 Hz, 1H), 7.12 (d, J = 16 Hz, 1H) ppm.

### Example 14

### (Synthesis of Compound H)

Compound H was synthesized according to the scheme below.

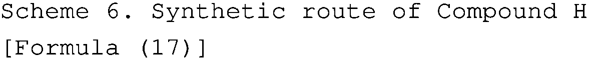

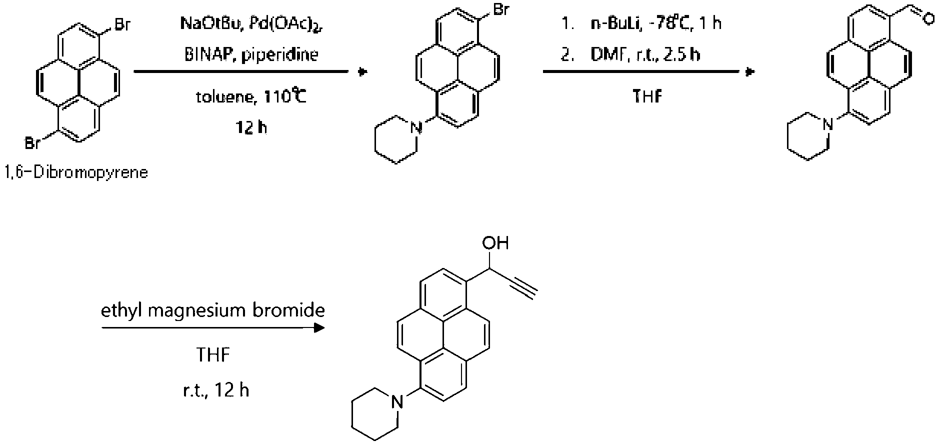

According to the above scheme, synthesis reactions were performed following Examples 1-4 as appropriate. As a result, the desired Compound H was obtained as a red solid (overall yield from starting materials: 68%; yield of the final step: 12%; Figure 18).
· H: ¹H NMR (500 MHz, CDCl₃, TMS): δ 9.42 (d, J = 9.3 Hz, 1H), 8.96 (d, J = 8.4 Hz, 1H), 8.58 (d, J = 9.1 Hz, 1H), 8.23 (d, J = 8.4 Hz, 1H), 8.22 (d, J = 9.3 Hz, 1H), 8.14 (d, J = 8.3 Hz, 1H), 8.06 (d, J = 9.1 Hz, 1H), 7.78 (d, J = 8.3 Hz, 1H), 3.52 (s, 1H), 3.24 (m, 4H), 1.96-1.92 (m, 4H), 1.73 (m, 2H) ppm.

### Example 15

### (Photophysical property measurements of Compounds F and H in solution)

UV-visible absorption spectra and fluorescence spectra of Compounds F and H obtained in Examples 12 and 14, respectively, were measured.

The UV-visible absorption spectra were measured using a spectrophotometer (JASCO V-670, JASCO Corporation). The fluorescence spectra were measured using a spectrofluorometer (JASCO FP-6600, JASCO Corporation). Fluorescence quantum yields were measured using an absolute PL quantum yield measurement system (Hamamatsu Photonics, C9920-02V). The concentration of each compound in each solvent was 5 µM. The results are shown in Figures 19 and 20, and in Tables 6 and 7. In addition, the spectra of Compound G are expected to be essentially the same as those of Compound F.

**[Table 6]**

| **Table 6. Photophysical parameters of Compound F in organic solvents** | | | |
|---|---|---|---|
| | Absorption maximum [nm] wavelength | Fluorescence maximum wavelength [nm] | Fluorescence quantum yield |
| 1,4-dioxane | 417 | 555 | 85 |
| DCM | 423 | 608 | 82 |
| DMSO | 426 | 644 | 73 |
| EtOH | 428 | 692 | 16 |

**[Table 7]**

| **Table 7. Photophysical parameters of Compound H in organic solvents** | | | |
|---|---|---|---|
| | Absorption Absorption maximum wavelength [nm] | Fluorescence maximum wavelength [nm] | Fluorescence quantum yield |
| 1,4-dioxane | 445 | 565 | 92 |
| DCM | 456 | 607 | 78 |
| DMSO | 457 | 632 | 61 |
| EtOH | 446 | 647 | 3 |

### Example 16

### (Water solubility test of Compounds A-C)

Method: As shown in Figure 21, when the dye aggregates in aqueous solution, the shape of the absorption spectrum changes before and after aggregation (see figure for an example using PK).

Note that 1-acetyl-6-piperidylpyrene (PK) is represented by the following chemical formula and is an SC compound (solvatochromic compound).

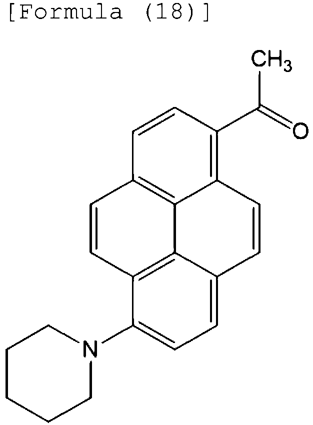

Figure 21 shows the absorption spectrum of PK (an analog of Compound A) in phosphate buffer. As indicated by the arrow, it can be seen that the spectral shape (e.g., absorption maximum) changes significantly at around 5 µM.

Using the above property, the water solubility of Compounds A-C was evaluated according to steps 1)-3) below.
1. A DMSO solution of the dye was added dropwise into 20 mM phosphate buffer solution (pH = 7.2), and the UV-visible absorption spectrum was measured. The DMSO concentration was adjusted to 0.5% or less.
2. Measurements were performed at various dye concentrations, and the spectral shapes were observed.
3. Based on the principle of UV-visible absorption, the maximum measurable concentration was defined as the point where the absorbance was approximately 2 (accurate spectra cannot be obtained when the absorbance approaches 3).

The results are shown in Figures 21-23. From these results, since no significant change in spectral shape was observed across the tested concentration ranges, it was determined that each compound exhibited water solubility of >150 µM.

### [Footnotes to Drawings]

FIG. 1 is a ¹H NMR spectrum (500 MHz, CDCl₃) of a mixture of Compounds 1a and 1b.
FIG. 2 is a ¹H NMR spectrum (500 MHz, CDCl₃) of Compound 2a.
FIG. 3 is a ¹H NMR spectrum (500 MHz, CDCl₃) of Compound 2b.
FIG. 4 is a ¹H NMR spectrum (500 MHz, DMSO-d₆) of Compound A.
FIG. 5 is a ¹H NMR spectrum (500 MHz, DMSO-d₆) of Compound B.
FIG. 6 shows the absorption spectrum (left) and fluorescence spectrum (right) of Compound A in organic solvents.
FIG. 7 shows the absorption spectrum (left) and fluorescence spectrum (right) of Compound B in organic solvents.
FIG. 8 shows fluorescence spectra of Compounds A (left) and B (right) in the presence of artificial lipid bilayers (liposomes).
FIG. 9 is a multiphoton fluorescence imaging image of mouse skin tissue (foot) (stratum spinosum, basal layer-dermal region).
FIG. 10 is a ^1H NMR spectrum (500 MHz, DMSO-d₆) of Compound C.
FIG. 11 is a ^1H NMR spectrum (500 MHz, DMSO-d₆) of Compound D.
FIG. 12 is a ^1H NMR spectrum (500 MHz, DMSO-d₆) of Compound E.
FIG. 13 shows the absorption spectrum (left) and fluorescence spectrum (right) of Compound C in organic solvents.
FIG. 14 shows the absorption spectrum (left) and fluorescence spectrum (right) of Compound D in organic solvents.
FIG. 15 shows the absorption spectrum (left) and fluorescence spectrum (right) of Compound E in organic solvents.
FIG. 16 is a ^1H NMR spectrum (500 MHz, DMSO-d₆) of Compound F.
FIG. 17 is a ^1H NMR spectrum (500 MHz, DMSO-d₆) of Compound G.
FIG. 18 is a ^1H NMR spectrum (500 MHz, CDCl₃) of Compound H.
FIG. 19 shows the absorption spectrum (left) and fluorescence spectrum (right) of Compound F in organic solvents.
FIG. 20 shows the absorption spectrum (left) and fluorescence spectrum (right) of Compound H in organic solvents.
FIG. 21 is an absorption spectrum of PK (an analogue of Compound A) in phosphate buffer solution.
FIG. 22 is an absorption spectrum of Compound A in phosphate buffer solution.
FIG. 23 is an absorption spectrum of Compound B in phosphate buffer solution.
FIG. 24 is an absorption spectrum of Compound C in phosphate buffer solution.

## Claims

1. A compound represented by the following Formula (1) or a salt thereof: 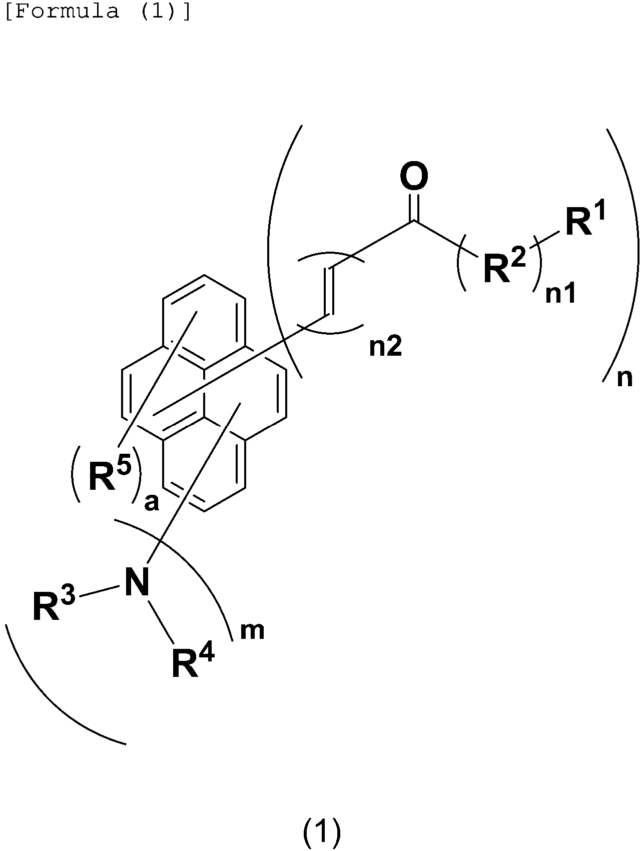 wherein,
R¹ is a substituted or unsubstituted C1-12 alkyl group, a C2-12 alkenyl group, or a C2-12 alkynyl group;
R² is a substituted or unsubstituted methylene group, wherein when a plurality of R² groups are present, they may be the same or different;
n¹ is an integer of 0-4, wherein when n¹ is 2 or more, the respective groups may be the same or different;
n is an integer of 1-4, wherein when n is 2 or more, the respective groups may be the same or different;
n² is an integer of 0-4, wherein when n² is 2 or more, the respective groups may be the same or different;
R³ and R⁴ are each independently selected from hydrogen atom, or a substituted or unsubstituted C1-6 alkyl group, C2-6 alkenyl group, or C2-6 alkynyl group, and R³ and R⁴ may together form a cyclic structure;
at least one of R¹, R³, or R⁴ includes at least one hydrophilic substituent selected from the group consisting of carboxyl group, hydroxyl group, sulfo group, amino group, amido group, halogen atom, and salts thereof, wherein when two or more hydrophilic substituents are present, the substituents may be the same or different;
m is an integer of 1-4, wherein when m is 2 or more, the respective groups may be the same or different;
R⁵ is each independently a C1-12 alkyl group, a C2-12 alkenyl group, a C2-12 alkynyl group, a C5-12 aryl group, a C5-12 heteroaryl group, a halogen atom, or a hydrophilic substituent comprising at least one atom selected from the group consisting of boron, nitrogen, oxygen, phosphorus, sulfur, and halogen; and
a is each independently an integer of 0-4, wherein when a is 2 or more, the respective R⁵ groups may be the same or different.

2. The compound or salt according to claim 1, wherein at least one R¹ is selected from a methyl group, an ethyl group, an n-propyl group, an i-propyl group, an n-butyl group, an i-butyl group, a sec-butyl group, a t-butyl group, an ethynyl group, a propargyl group, or a substituted or unsubstituted 1,2,3-triazole or 1,2,4-triazole group having a C1-4 alkyl group.

3. The compound or salt according to claim 1, wherein R³ and R⁴ are each independently selected from hydrogen atom, a carboxyl group, a hydroxyl group, a sulfo group, an amino group, an amido group, a halogen atom, or a substituted or unsubstituted methyl group, ethyl group, n-propyl group, i-propyl group, n-butyl group, i-butyl group, sec-butyl group, or t-butyl group.

4. The compound or salt according to claim 1, wherein at least one pair of R³ and R⁴ together form a pyrrolidine ring or a piperidine ring.

5. The compound or salt according to claim 1, wherein n is 1 or 2.

6. The compound or salt according to claim 1, wherein n¹ is 1 or 2.

7. The compound or salt according to claim 1, wherein m is 1 or 2.

8. The compound or salt according to claim 1, wherein R⁵ is each independently selected from a methyl group, an ethyl group, a hydroxyl group, or an amino group.

9. The compound or salt according to claim 1, wherein the maximum absorption wavelength is 300-550 nm in 20 mM phosphate buffer (pH 7.4) at 25 °C.

10. The compound or salt according to claim 1, wherein the molecular weight is 700 or less.

11. A fluorescent dye comprising the compound or salt according to any one of claims 1-10.

12. The fluorescent dye according to claim 11, for morphological detection of cells and/or tissues.

13. The fluorescent dye according to claim 11, for morphological detection of tumor cells.

14. The fluorescent dye according to claim 11, for morphological detection of skin cancer cells.

15. The fluorescent dye according to claim 11, for staining or visualization of biological samples.

16. The fluorescent dye according to claim 11, for fluorescent imaging.

17. The fluorescent dye according to claim 11, for in vivo observation.

18. The fluorescent dye according to claim 11, for transdermal absorption.

19. A kit comprising the compound or salt according to any one of claims 1-10.

20. A method for detecting cells, comprising a step (1) of staining the cells with the fluorescent dye comprising the compound or salt according to any one of claims 1-10.

21. The method for detecting cells according to claim 20, further comprising a step (2) of evaluation using fluorescence spectra measured in two or more solvents after step (1).

22. The method for detecting cells according to claim 21, wherein step (2) is performed by fluorescent imaging.

23. A material for staining lipid bilayers, oil droplets, or protein aggregates present in cells or modeling a cellular environment, comprising the compound or salt according to any one of claims 1-10.
